# EUROPEAN PATENT SPECIFICATION

(11) **EP 4 013 834 B1**
(45) Date of publication and mention of the grant of the patent: **19.02.2025**
(21) Application number: 20815710.7
(22) Date of filing: 09.10.2020
(51) Int. Cl.: C09K 11/06, C08F 220/56, C08F 220/58, C08F 220/60, G01N 21/64, G01N 33/533, G01N 33/58, C08L 33/26

(54) **FLUORESCENT POLYMER, FLUORESCENT PROBE AND CONJUGATION KIT FOR ADVANCED FUNCTIONAL ANALYSIS OF CELLS IN HAEMATOLOGY, IMMUNOLOGY AND MICROBIOLOGY, METHOD OF PREPARATION AND USE THEREOF**
FLUORESZIERENDES POLYMER, FLUORESZIERENDE SONDE UND KONJUGATIONSKIT FÜR DIE FORTGESCHRITTENE FUNKTIONELLE ANALYSE VON ZELLEN IN DER HÄMATOLOGIE, IMMUNOLOGIE UND MIKROBIOLOGIE, VERFAHREN ZUR HERSTELLUNG UND VERWENDUNG DAVON
POLYMÈRE FLUORESCENT, SONDE FLUORESCENTE ET KIT DE CONJUGAISON POUR ANALYSE FONCTIONNELLE AVANCÉE DE CELLULES EN HÉMATOLOGIE, IMMUNOLOGIE ET MICROBIOLOGIE, LEUR PROCÉDÉ DE PRÉPARATION ET LEUR UTILISATION

(30) Priority: 09.10.2019 CZ 201936721 U; 09.10.2019 CZ 20190627
(43) Date of publication of application: 22.06.2022
(73) Proprietor: I.T.A.-Intertact s.r.o., 110 00 Praha 1 (CZ); Ustav Makromolekularni Chemie AV CR, v.v.i., 162 06 Praha 6 (CZ); Univerzita Karlova, 116 36 Praha 1 (CZ)
(72) Inventor: HOVORKA, Ondrej, 267 28 Svinare (CZ); ETRYCH, Tomas, 252 10 Klinec (CZ); KALINA, Tomas, 150 00 Praha 5 (CZ)
(74) Representative: Harber IP s.r.o.
(86) International application number: PCT/CZ2020/050078
(87) International publication number: WO 2021/069000

(56) References cited:
- US-A1- 2017 014 531
- ETRYCH T ET AL: "HPMA copolymer conjugates with reduced anti-CD20 antibody for cell-specific drug targeting. I. Synthesis and in vitro evaluation of binding efficacy and cytostatic activity", JOURNAL OF CONTROLLED RELEASE, ELSEVIER, AMSTERDAM, NL, vol. 140, no. 1, 16 November 2009 (2009-11-16), pages 18 - 26, XP026691078, ISSN: 0168-3659, [retrieved on 20090724], DOI: 10.1016/J.JCONREL.2009.07.011

## Description

### Field of Art

The present invention relates to a fluorescent polymer composed of a polymeric carrier with side chains with fluorophores, enabling highly efficient labelling of antibodies. The invention further relates to a fluorescent probe, a construct composed of a polymeric carrier with fluorophores (fluorescent polymer) and a protein structure, for example, an antibody. Its use focuses on flow cytometry, microscopic analysis and other imaging techniques using fluorophore-labelled antibodies for diagnostics, quality control and science and research.

### Background Art

The use of fluorescent probes in immunology, haematology and microbiology has grown enormously in the last two decades. These are basic methods used daily in workplaces around the world, from basic research to hospital laboratories and analytical departments. Commercially available, fluorescently labelled antibodies are used in clinical diagnostics for analysing the state of the immune system of patients, in haemato-oncology for the diagnosis of malignant disease, in pathology for the detection of changes and disorders and changes in tissues. They are also used for hundreds of applications in science and research from physiological and immunological to evolutionary, anatomical and developmental analyses.

Hundreds of different antibodies against various biological targets are currently available on the market, either labelled with fluorophores or in the form of so-called purifiers - i.e. without covalently bound fluorescent labels, which must be subsequently detected using so-called labelled secondary antibodies.

Etrych et al., HPMA copolymer conjugates with reduced anti-CD20 antibody for cell-specific drug targeting. I. Synthesis and in vitro evaluation of binding efficacy and cytostatic activity; Journal of Controlled Release, vol. 140, no. 1 (2009), p. 18-26, describes semitelechelic polymers having a pHPMA backbone to which an anticancer drug doxorubicine is bound via an oligopeptide linker degradable by lysosomal enzymes or via a pH-labile hydrazone linkage. The polymer backbone has a reactive thiazolidine-2-thione amide or maleimide end functionality, which enables to target the polymers to anti-CD20 monoclonal antibody forming a star-shaped structure with central antibody surrounded by hydrophilic polymer chains. The star-shaped structure targets cancer cells wherein degradation of the pH-labile or enzyme-labile linker hydrolyses releasing doxorubicin at the site of action.

US 2017/014531 A1 discloses fluorescent polymers for theranostic applications, designed to generate a fluorescent signal in response to a chemical event (upon contacting an analyte - an enzyme - over-expressed in a diseased tissue). The enzyme degrades the peptidic binding of the fluorophore inside the polymer, the fluorophore is thus released from the polymer and the fluorescence in a diseased tissue is enhanced. The peptidic binding of fluorophore is designed to undergo enzymatic degradation.

The growth of bioinformatics and technological progress in the design of detection systems (whether flow cytometers, imaging systems or chip and membrane readers) led to the massive development of multiplex analysis, allowing to significantly multiply the number of features detected simultaneously in individual experiments. This development was also reflected in need for new fluorophores to supplement the spectrum of fluorescent labels used. Hundreds of different fluorophores with different absorption and emission characteristics and fluorescence intensity derived from various modifications of other starting chemical structures are currently available on the market.

In the multiplex analysis, a panel of labelled antibodies should be used, each carrying its fluorophore distinguishable by its spectral profile (absorption and emission maxima) from all other antibodies in the panel. When setting up an antibody diagnostic/analytical panel in the case of more extensive analyses (10 antibodies and more), users encounter the problem of unavailability of specific labelled commercial antibodies against the minor, little experimentally used or newly defined markers which they require to analyse.

Many products are available on the market, allowing users to conjugate a particular fluorophore to its unlabelled antibody. The problem with these kits, however, is the small choice of fluorophores - most of them spectrally overlap with other commonly available fluorophores, and especially the low level of labelling. The results are fluorescently labelled antibodies, but their use is limited, and low-expression markers cannot be detected with these products at all. Spectrally 'interesting' fluorophores emitting in regions where they can be easily distinguished, then usually have a low fluorescence quantum yield and directly labelled conjugates are difficult to distinguish. Polymer fluorescent probes amplify the number of fluorescent molecules and thus significantly amplify the detectable signal. It is, therefore, possible to use non-traditional, spectrally and functionally unique fluorophores and combinations of fluorophores.

### Summary of the Invention

The present invention relates to a fluorescent polymer, a fluorescent probe and a conjugation kit for rapid labelling of antibodies, proteins and other structures with a suitably modified surface. The fluorescent polymer and conjugation kit of the present invention allows the labelling of own appropriately modified structures by introducing on their surface semitelechelic copolymer chains having fluorescent label molecules (fluorophores) attached to them. The reactive end groups of the semitelechelic polymer chain allow a single point binding of the fluorescent polymer to the protein structure and thus the fluorescent labelling of the antibody.

Thanks to this approach, up to several times higher amounts of fluorophore can be introduced into a suitable protein structure than with conventional commercially available kits. The resulting fluorescent probe allows the amplification of a signal which reaches and exceeds the intensity of labelling using commercially available biomolecules and nanoparticles. A suitable protein structure can be labelled using the conjugation kit of the present invention in units of minutes and with a minimum number of steps by a fluorescently labelled hydrophilic copolymer, e.g. based on N-(2-hydroxypropyl)methacrylamide (HPMA), to prepare a polymeric fluorescent probe. Along the chain of this copolymer, functional groups are introduced to which suitable fluorophore derivatives can be attached; at one of its ends, they contain a reactive group for single-point grafting onto the protein structure. Another field of application of such systems according to the present invention is the possibility of creating own combination of several fluorophores bound in several different desired ratios to the structure of interest to create a precisely defined fluorescence pattern that is unique for each particular structure. The resolution of the intensity ratios gives rise to a unique fluorescent signature, where it is then possible to distinguish up to 8 different spectral combinations in two channels, and thus to distinguish up to eight types of cell subtypes (subsets). These 'barcoding' spectral technologies can be used to distinguish between different samples, which will first be fluorescently 'coded' and then mixed and labelled all at once, which eliminates differences in labelling techniques and allows direct comparison of the expressions of the monitored markers. The 'barcoding' marker occupies only two spectral bands but allows up to eight different samples to be distinguished. The remaining part of the detection spectrum can be used for other evaluated (diagnostic) markers. In addition, two or more fluorophores reacting differently to physicochemical or biological parameters of the microenvironment of labelled cells, e.g. pH, temperature, ion concentration, enzyme activity, etc., can be combined. One of the used markers is stable in the environment and allows normalising the signal from different cells, and other signals can be variable. The ratio of the normalised and sensitive signal can then be used to determine the parameters of the environment directly. The whole system according to the present invention thus makes it possible to increase the detectability of the monitored markers in one cell and at the same time significantly accelerate the preparation of fluorescently labelled structures with a suitably modified surface.

The object of the present invention is a fluorescent polymer for the rapid labelling of antibodies, proteins and other structures with a suitably modified surface, comprising a linear semitelechelic statistical copolymer to which at least one fluorescent label (fluorophore) is attached in an amount from 0.4 to 12 mol %, preferably from 0.5 to 6 mol %, based on the number of monomer units, wherein the linear semitelechelic statistical polymer is based on polyacrylamide copolymers, polymethacrylamide copolymers, polyacrylate copolymers or polymethacrylate copolymers, preferably comprises copolymers of poly(*N*-(2-hydroxypropyl)methacrylamide, wherein in the linear semitelechelic statistical copolymer from 0.4 to 12 mol % of monomer units is statistically replaced by monomer units of general formula (I) 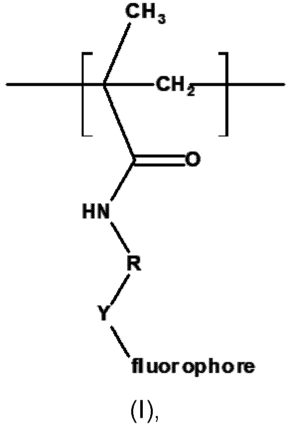 wherein
R is selected from the group consisting of a straight or branched carbon alkylenyl chain with a number of carbons from 1 to 6 ((C1 to C6)alkylenyl); wherein R may be further substituted with one or more identical or different natural amino acid side chains; preferably these chains are methyl, isopropyl, isobutyl, -CH(CH₃)(CH₂CH₃), -CH₂OH, -CH(OH)(CH₃), -CH₂-(C₆H₄)OH, -(CH₂)₂-S-CH₃, -CH₂SH, -(CH₂)₄-NH₂, -CH₂COOH, -CH₂C(O)NH₂, -(CH₂)₂COOH, -(CH₂)₂C(O)NH₂, -(CH₂)₃NH-C(=NH)(NH₂), benzyl;
Y is selected from the group consisting of a bond, -C(=O)-NH-, -NH-C(=O)-, -NH-C(=S)-NH-; wherein the molecular weight Mₙ of the linear semitelechelic copolymer is in the range of from 10,000 to 100,000 g/mol, preferably in the range of from 15,000 to 40,000 g/mol (corresponding to from 100 to 280 monomer units), more preferably the molecular weight is in the range of from 20,000 to 30,000 g/mol (corresponding to from 134 to 210 monomer units);
and wherein the fluorescent label (fluorophore or amino derivative, NCS derivative or *N-*hydroxysuccinimide derivative thereof) has a molecular weight in the range of from 350 to 1,500 g/mol, an excitation wavelength in the range of from 300 to 850 nm and an emission wavelength in the range of from 350 to 1,200 nm and is covalently bound to a monomer unit of general formula I of a linear semitelechelic copolymer by the reaction of its primary amino group, thiocyanate or N-hydroxysuccinimide ester, the groups formed after fluorophore attachment being subsequently part of group Y;
and wherein the fluorescent polymer contains at one end of the polymer chain a functional group for attachment to a protein structure (e.g. by single-point grafting), which functional group is selected from the group comprising esters, preferably N-hydroxysuccinimide ester or C1-C4 alkyl esters, amides, preferably thiozolidine-2-thione amide, maleimide, azide, propargyl, preferably the functional group is azide or maleimide.

By natural amino acids are meant histidine, isoleucine, leucine, lysine, methionine, phenylalanine, threonine, tryptophan, valine, arginine, cysteine, glutamine, glycine, proline, tyrosine, alanine, aspartic acid, asparagine, glutamic acid, serine, selenocysteine. The side chains are chains attached to the alpha-carbon of the amino acid.

In one preferred embodiment, the natural amino acid side chain is selected from the group comprising methyl, isopropyl, isobutyl, -CH(CH₃)(CH₂CH₃), -CH₂OH, -CH(OH)(CH₃), -CH₂-(C₆H₄)OH, -(CH₂)₂-S-CH₃, -CH₂SH, -(CH₂)₄-NH₂, -CH₂COOH, -CH₂C(O)NH₂, -(CH₂)₂COOH, -(CH₂)₂C(O)NH₂, -(CH₂)₃NH-C(=NH)(NH₂), benzyl.

The fluorescent polymer may further contain up to 11.6 mol % of monomer units of general formula (II), based on the number of monomer units, 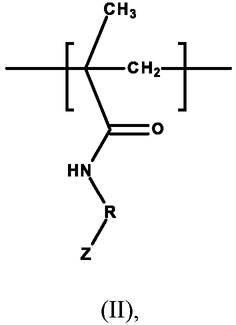 wherein R is as defined above and Z is selected from the group comprising -C(=O)-NH-(CH₂)ₐ-CH₂(OH); -C(=O)-NH-(CH₂)_{b}-CH(OH)-CH₃; -C(=O)-NH-(CH₂)_{b}-CH(OH)-(CH₂)_{c}-CH₃; wherein *a* is an integer from 0 to 4, *b* is an integer from 0 to 3 and c is from 1 to 4; and -NH-C(=O)-CH₃,
wherein in total the monomer units of general formula (I) and (II) in the fluorescent polymer are at most 12 mol %, based on the number of monomer units.

In one preferred embodiment, R is selected from the group comprising ethan-1,2-diyl (-CH₂-CH₂-); propane-1,3-diyl (-CH₂-CH₂-CH₂-); hexane-1,6-diyl (-(CH₂)₆-).

A linear semitelechelic copolymer is a statistical copolymer formed by radical polymerisation. Thus, the end groups of the resulting linear copolymer contain portions of polymerisation initiator molecules (the initiator may be, for example, 2,2'-azobis[N-(2-carboxyethyl)-2-methylpropionamidine] (V-70)) and a transfer agent (the transfer agent may be, for example, *N-*(3-azidopropyl)-4-ethylsulphanylcarbothioylsulphanyl-4-methylpentanamide (CTA-N₃)), or their derivatives formed by reaction with, for example, 2,2'-azobisisobutyronitrile (AIBN). Thus, the end groups of the copolymer contain an azide group, which can be subsequently modified by reaction with dibenzocyclooctyne-maleimide (DBCO-MI) to another maleimide reactive functional group, by reaction with dibenzocyclooctyne-carboxylic acid (DBCO-Cbx), which is subsequently modified with thiazolidine-2-thione to a thiazolidine-2-thione reactive functional group, by reaction with the dibenzocyclooctyne-N-hydroxysuccinimide ester (DBCO-NHS) to an N-hydroxysuccinimide ester reactive functional group. Unlike conventional radical polymerisation with an initiator, which in principle does not allow the formation of a semitelechelic copolymer (does not allow the introduction of reactive end groups at the end of the chain, because no transfer agent is used in the polymerisation), to enable this polymer to be grafted onto the protein in a single point, the presence of a so-called transfer agent is necessary, which introduces a suitable reactive group into the polymer structure. An appropriate reaction is, for example, the RAFT radical polymerisation.

In one preferred embodiment, the linear semitelechelic copolymer is poly(N-(2-hydroxypropyl) methacrylamide), the resulting fluorescent polymer structure in this embodiment comprises the general formula (III), 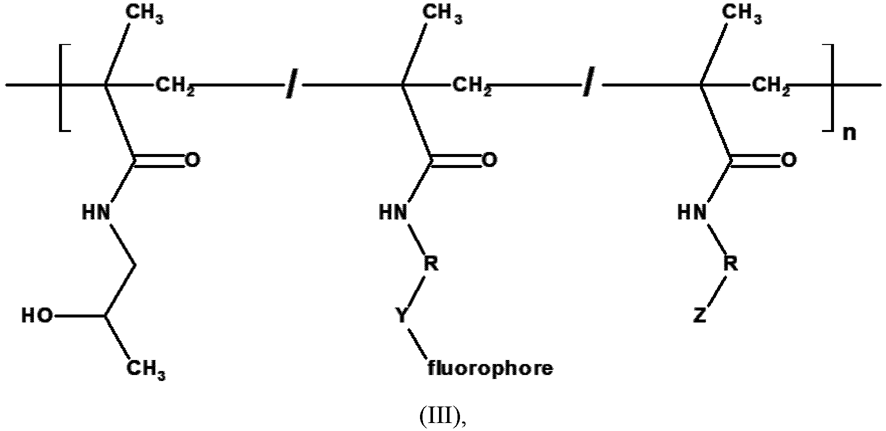 wherein R, Y and Z are as defined above, n is an integer in the range of from 100 to 280; the proportion of units of general formula (I) in this linear statistical copolymer is from 0.4 to 12 mol %, based on the number of monomer units, and the proportion of units of general formula (II) in this linear statistical copolymer is from 0 to 11.6 mol %, based on the number of monomer units, the total monomer units of the general formulas (I) and (II) being at most 12 mol %, based on the number of monomer units. The end groups of the resulting linear copolymer of general formula (III) contain portions of the polymerisation initiator and transfer agent molecules, and a functional group for binding to a protein structure, preferably N-hydroxysuccinimide ester, thiozolidine-2-thione amide, maleimide, azide, or propargyl.

In one embodiment, the semitelechelic copolymers that are subsequently part of the fluorescent polymer of the present invention contain -N₃ end groups introduced during the polymerisation reaction which can be used to introduce maleimide for conjugation to a protein structure with a suitably modified surface.

The fluorescent label (fluorophore) is selected according to excitation and emission wavelengths, wherein the excitation wavelengths are in the range of from 300 to 850 nm, and the emission wavelengths are in the range of from 350 to 1,200 nm. By low-molecular-weight fluorophore is meant a fluorophore having a molecular weight in the range of from 350 to 1,500 g/mol. Preferably, the fluorophore or derivative thereof contains a functional group, selected from an amino group, an isothiocyanate group and an N-hydroxysuccinimide group, for attachment to the amino or TT group of the linear copolymer side chain. Preferably, the fluorescent label is selected from the group comprising fluorescein, fluorescein isothiocyanate (FITC), derivatives of ruthenium-bipyridine complexes Ru(bpy)₃, in particular bis(2,2'-bipyridine)-[4-(4'-methyl-2,2'-bipyridin-4-yl)butan-1-aminium ruthenium tris(perchlorate) (RUB-C4), cyanines, especially Cyanine3 or Sulfo-Cyanine7.5, also 3,6-diamino-9-[4-(2-aminoethylcarbamoyl)-2-carboxyfenyl]-5-(3-sulphonatopropylsulphamoyl)xanthene-10-ium-4-sulphonate (Dy-490), (2E)-1-[6-(2,5-dioxopyrro-lidin-1-yl)oxy-6-oxohexyl]-2-[(2E,4E)-5-[1-(2-methoxyethyl)-3,3-dimethyl-5-sulphonato-indol-1-ium-2-yl]penta-2,4-dienylidene]-3,3-dimethyl-indoline-5-sulphonate (Dy-647P1), 3-[4-[(E)-2-[6-[6-(2-aminoethylamino)-6-oxohexoxy]-1,3-benzoxazol-2-yl]vinyl]-1-pyridyl]propane-1-sulphonate (Dy-396XL), 3-[4-[6-[[6-(2,5-dioxopyrrolidin-1-yl)oxy-6-oxohexyl]carbamoylamino]-1,3-benzoxazol-2-yl]pyridin-1-ium-1-yl]propane-1-sulphonate (Dy-395XL), 3-[[6-(2-azaniumyl-ethylamino)-6-oxohexyl]carbamoyl]-8-chloro-7-hydroxy-2-oxo-chromene-6-sulphonate (Dy-410), 3-[4-[7-[[6-(2-aminoethylamino)-6-oxohexyl]-ethyl-amino]-2-oxo-chromen-3-yl]pyridin-1-ium-1-yl]propane-1-sulphonate (Dy-485XL), 1-[6-(2-aminoethylamino)-6-oxo-hexyl]-6-[(E)-2-[7-(diethylamino)-2-oxo-chromen-3-yl]vinyl]pyridin-1-ium-3-sulphonate (Dy-480), 1-[6-(2-aminoethylamino)-6-oxo-hexyl]-4-[(E)-2-[7-(diethylamino)-2-oxo-chromen-3-yl]vinyl]pyridin-1-ium-3-sulphonate (Dy-520XL), (2E)-2-[(E)-3-(7-amino-2-*terc*-butyl-chromenylium-4-yl) prop-2-enylidene]-1-[6-(2-aminoethylamino)-6-oxohexyl]-3,3-dimethyl-indoline-5-sulphonate (Dy-615), (2Z)-3-[4-(2-aminoethylamino)-4-oxobutyl]-2-[(E)-3-[4-*terc*-butyl-7-[ethyl(3-sulpho-natopropyl)amino]chromenylium-2-yl]prop-2-enylidene]-3-methyl-1-(3-sulphonatopropyl)indo-line-5-sulphonate (Dy-682) and (2Z)-3-[4-(2-aminoethylamino)-4-oxo-butyl]-2-[(E)-3-[7-(diethylamino)-3-methyl-4-phenyl-chromenylium-2-yl]prop-2-enylidene]-3-methyl-1-(3-sulpho-natopropyl)indoline-5-sulphonate (Dy-701), or their derivatives containing a functional group selected from an amino group, an isothiocyanate group and an N-hydroxysuccinimide group.

In one preferred embodiment, the fluorescent polymer of the present invention comprises at least two different fluorescent labels, for example, combinations Dy-490 and Dy-647P1, Dy-396XL and Dy-647P1, Dy-560 and Dy-647P1, Dy-396XL and Dy-560, Dy-490 and Dy-480XL, Dy-396XL and Dy-480XL and a variety of other, spectrally non-overlapping fluorophores. These different fluorescent labels can be bound to one fluorescent polymer in different molar ratios, preferably from 1:1 to 1:15, resulting in a different ratio of fluorescence intensities. More preferably, fluorophores with different excitation and emission wavelengths are combined in one polymer.

Another object of the present invention is a fluorescent probe comprising at least one fluorescent polymer according to the present invention, and further comprising a protein structure selected from the group of a peptide having an amino acid number ranging from 10 to 200, a protein, a lipoprotein, an antibody, an enzyme, a hormone, a receptor, a structural protein, a transport protein, a cell signalling pathway protein, a synthetic protein, wherein the protein structure may be modified before conjugation with the fluorescent polymer to contain at least one group selected from -NH₂, -SH, sDBCO (sulphodibenzocyclooctyne group), DBCO (dibenzocyclooctyne group), azide, to which the fluorescent polymer is covalently bound via a functional group, preferably selected from N-hydroxysuccinimide ester, thiozolidine-2-thione amide, maleimide, azide, or propargyl, contained at the end of the linear polymer chain of the fluorescent polymer. This functional group is covalently bound to an -NH₂ group (e.g. lysine residues), -SH group (introduced, e.g. by reduction of disulphide bonds), or to sDBCO, DBCO, and azide groups introduced onto the protein structure to form a covalent bond.

In one preferred embodiment, the fluorescent probe comprises a monoclonal antibody to which at least one fluorescent polymer according to the present invention is covalently bound. The monoclonal antibody may be, for example, anti-CD20, anti-CD3, anti-CD4, anti-CD8, anti-CD71, anti-MHCII, anti-CD45, anti-JNK. Monoclonal antibodies have a monovalent affinity and bind specifically to a single epitope of an antigen.

The monoclonal antibody contains a heavy chain which is type-specific and two light chains which have an antigen-binding site. The fluorescent polymer is covalently bound either to the light chain of the monoclonal antibody at sites where the monoclonal antibody contains disulphide bonds which are reduced before binding to the fluorescent polymer, or along the entire antibody structure at sites containing free amine groups (e.g. from lysine side chains). The reduction of disulphide bonds can be carried out, for example, by treatment with dithiothreitol (DTT), *tris*(2-carboxyethyl)phosphine (TCEP), *β*-mercaptoethanol or glutathione. The monoclonal antibody thus prepared can be immediately conjugated to the fluorescent polymer of the present invention, wherein the SH groups formed by reducing the disulphide bonds of the monoclonal antibody react with a linear polymer chain end group of the fluorescent polymer, e.g., a maleimide group, or the SH groups of the antibody can be further converted by reaction with sDBCO-MI (sulphodibenzocyclooctyne maleimide) to sDBCO groups, or the free NH₂ groups of the antibody can be converted to DBCO groups by reaction with DBCO-NHS (dibenzocyclooctyne-N-hydroxysuccinimide ester). Alternatively, NH₂ groups of the antibody can be converted to azide groups by reaction with NHS-Azide. The resulting DBCO, sDBCO or azide groups of the antibody are then subsequently reacted with a linear polymer chain end group of the fluorescent polymer, preferably selected from the group comprising azide, or propargyl, to form a covalent bond between the fluorescent polymer and the antibody.

The method of preparing the fluorescent probe (the fluorescent polymer with a bound protein structure) comprises the following steps:
a) Providing the monomers of the linear semitelechelic copolymer;
b) Polymerisation of the monomers of the linear semitelechelic copolymer;
c) Attaching a fluorescent label to the linear semitelechelic copolymer to form a fluorescent polymer;
d) Optional modification of the protein structure;
e) Binding the protein structure to the fluorescent polymer.

Step a) of providing the monomers of a linear semitelechelic copolymer includes providing N-(2-hydroxypropyl)methacrylamide (HPMA), acrylamide or derivatives thereof, methacrylamide or derivatives thereof, acrylate or derivatives thereof or methacrylate or derivatives thereof, and providing monomers of the general formula (IV) 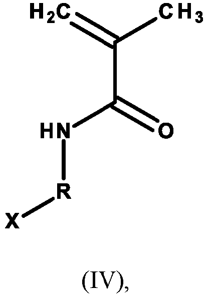
wherein R is selected from the group consisting of a straight or branched carbon alkylene chain with a number of carbons from 1 to 6 ((C1 to C6)alkylene); wherein R may be further substituted with one or more of the same or different natural amino acid side chains;
   and
X is a carbonyl-thiazoline-2-thione group (TT) or an NH₂ group.

The amino group may be protected with a protecting group, for example, a tert-butoxycarbonyl (Boc) protecting group. Carbonyl means a -C(= O)- group. N-(2-hydroxypropyl)methacrylamide (HPMA) and other acrylamide, methacrylamide, acrylate and methacrylate type monomers are commercially available.

Compounds of formula (IV) were either obtained from commercially available sources, for example, methacryolyl-6-aminoropylamine protected on amine groups by tert-butyloxycarbonyl groups (MA-Pr-NH-Boc), or were prepared according to procedures mentioned in the literature (Pola R., Parnica J., Zuska K., Böhmová E., Filipová M., Pechar M., Pankrác J., Mucksová J., Kalina J., Trefil P., Šefc L., Větvička D., Poučková P., Bouček J., Janoušková O., Etrych T. *Multifunct. Mater.* 2019, 2: 024004).

Step b) of polymerisation of the monomers of the linear semitelechelic copolymer is carried out by controlled RAFT radical polymerisation of the monomers from step a) with a content of 0.4 to 12 mol % of the monomer of general formula (IV), and at least 88 mol % (88 to 99.6 mol %) of monomer units selected from the group comprising *N*-(2-hydroxypropyl) methacrylamide (HPMA) and other monomers based on acrylamides, methacrylamides, acrylates and methacrylates, preferably selected from the group comprising HPMA, acrylamide, methacrylamide. The reaction takes place at a temperature in the range of from 30 to 100 °C, preferably from 40 to 80 °C, and a solvent preferably selected from the group comprising dimethylsulphoxide, dimethylacetamide, dimethylformamide, methanol, ethanol, dioxane, tetrahydrofuran, propanol, tert-butanol or mixtures thereof.

The reaction is initiated by an initiator, preferably selected from the group comprising V-70 and ABIK-N₃, wherein V-70 is 2,2'-azobis[*N*-(2-carboxyethyl)-2-methylpropionamidine] and ABIK-N₃ is *N*-(3-azidopropyl)-4-[(Z)-[4-(3-azidopropylamino)-1-cyano-1-methyl-4-oxobutyl] azo]-4-cyano-pentanamide, optionally in the presence of a transfer agent, preferably CTA-N₃, wherein CTA-N₃ is *N*-(3-azidopropyl)-4-ethylsulphanylcarbothioylsulphanyl-4-methyl-pentanamide. The polymeric carrier is then terminated by a residue from the radical formed by the decomposition of the initiator used.

The resulting linear semitelechelic copolymer preferably contains an -N₃ end group; wherein the - N₃ end group of this resulting semitelechelic copolymer may be further reacted with suitable maleimide derivatives, preferably selected from the group of dibenzocyclooctyne-amine (DBCO-NH₂) or propargyl derivatives.

The prepared linear semitelechelic copolymer from step b) may be further subjected to removal of protecting groups protecting the amine groups of the side chains (e.g. Boc groups). Deprotection can be accomplished by established procedures known to those skilled in the art, for example, removal of the Boc group with trifluoroacetic acid or by heating the copolymer in water. The resulting copolymer/product can be stored without the risk of decomposition. The types of reactions which are preferably used to prepare the semitelechelic copolymers of the present invention are the RAFT polymerisation (using a pre-prepared transfer agent, e.g., CTA-N₃) and the 'click reaction' (e.g. when using dibenzocyclooctyne, propargyl, or azide end groups for semitelechelic copolymers).

Step c) of binding a fluorescent label to the linear semitelechelic copolymer to form a fluorescent polymer is accomplished by conjugating the free amine groups or thiazolidine-2-thione (TT) groups of the monomer units of general formula (IV) of the linear semitelechelic copolymer described above to a low-molecular-weight fluorescent label (fluorophore), which contains suitable reactive groups (e.g. amine or SCN) and which may be used in free form or the form of a salt with an acid, e.g. HCl; wherein the low-molecular-weight fluorophore is a fluorophore having a molecular weight in the range of from 350 to 1,500 g/mol. The fluorophore is selected according to excitation and emission wavelengths, wherein the excitation wavelengths range from 300 to 850 nm, and the emission wavelengths range from 350 to 1,200 nm; wherein the molecule of the low-molecular-weight fluorophore is bound to the linear semitelechelic copolymer by an amide or thioamide bond;
- any unreacted thiazolidine-2-thione groups can be removed by reaction with amino alcohol, selected from the group comprising NH₂-(CH₂)ₐ-CH₂(OH); NH₂-(CH₂)_{b}-CH(OH)-CH₃; NH₂-(CH₂)_{b}-CH(OH)-(CH₂)_{c}-CH₃; wherein *a* is an integer from 0 to 4, *b* is an integer from 0 to 3 and c is from 1 to 4, preferably with 1-aminopropan-2-ol, and/or the unreacted NH₂ groups can be removed by reaction with acetylthiazolidine-2-thione;
- an optional step of purification by column chromatography and lyophilisation of the final product from the previous step.

Alternatively, the fluorescent polymer can then be subjected to further reactions leading to a change of the reactive group at the end of the semitelechelic chain. An example is the click reaction of an azide group with dibenzocyclooctyne maleimide to form a fluorescent polymer with a maleimide functional group at one end of the linear polymer chain;
or the click reaction of an azide group with dibenzocyclooctyne-carboxylic acid and subsequently with thiazolidine-2-thione to form a fluorescent polymer with a thiazolidine-2-thione functional group at one end of the linear polymer chain;
or the click reaction of an azide group with a dibenzocyclooctyne-N-hydroxysuccinimide ester to form a fluorescent polymer with an N-hydroxysuccinimide ester functional group at one end of the linear polymer chain.

The optional step d) is the modification of a protein structure selected from the group of peptide, protein, lipoprotein, antibody, enzyme, hormone, receptor, structural protein, transport protein, cell signalling pathway protein, synthetic protein, preferably such protein structure that contains disulphide bonds; wherein the modification consists in reducing the disulphide bonds with a reducing agent selected from the group of dithiothreitol (DTT), tris(2-carboxyethyl)phosphine (TCEP), *β*-mercaptoethanol, glutathione, preferably DTT, in an aqueous buffer (pH 6.5 to 7.5) at room temperature to form SH-groups in the protein structure. These SH groups can then be further modified with sDBCO-MI (sulphodibenzocyclooctyne-maleimide) to sDBCO groups, or free NH₂ groups of the antibody (e.g. amino groups of lysine residues) can be modified to DBCO or azide groups by reaction with DBCO-NHS (dibenzocyclooctyne-*N*-hydroxysuccinimide ester) or NHS-azide. The resulting -SH, azide, DBCO and sDBCO groups or the original -NH₂ groups of the antibody are subsequently reacted in step e) with an end group of the linear polymer chain of the fluorescent polymer, preferably selected from the group consisting of *N*-hydroxysuccinimide ester, thiozolidine-2-thione amide, maleimide propargyl or azide, to form a covalent bond between the fluorescent polymer and the antibody.

The step e) of binding the protein structure to the fluorescent polymer - the step of conjugating of at least one fluorescent polymer according to the present invention and the protein structure or protein structure with introduced sulphhydryl (-SH), azide, sulphodibenzocyclooctyne (sDBCO) or dibenzocyclooctyne (DBCO) groups prepared in the previous step, wherein the reaction takes place at room temperature in aqueous buffer (pH 6.5 to 7.5); the end group of the fluorescent polymer reacts with -NH₂, -SH, propargyl, sDBCO or DBCO groups present on the protein structure in the order of minutes and in high yield, and the resulting conjugate retains its unaffected biological effect;
- optionally a step of purification by column chromatography and lyophilisation of the final product from the previous step.

In one preferred embodiment, at least two different fluorescent polymers are bound to the protein structure, preferably with different fluorophores.

The intermediate product for preparing the fluorescent polymer according to the present invention is, for example, a linear semitelechelic copolymer containing poly(*N*-(2-hydroxypropyl) methacrylamide) in which from 0.4 to 12 mol % of monomer units are replaced by a monomer unit of general formula (I),
wherein
R is selected from the group consisting of a straight or branched carbon alkylene chain with a number of carbons from 1 to 6 ((C1 to C6)alkylene); wherein R may be further substituted with one or more of the same or different natural amino acid side chains; preferably these chains are methyl, isopropyl, isobutyl, -CH(CH₃)(CH₂CH₃), -CH₂OH, -CH(OH)(CH₃), -CH₂-(C₆H₄)OH, - (CH₂)₂-S-CH₃, -CH₂SH, -(CH₂)₄-NH₂, -CH₂COOH, -CH₂C(O)NH₂, -(CH₂)₂COOH, - (CH₂)₂C(O)NH₂, -(CH₂)₃NH-C(=NH)(NH₂), benzyl;
Y is selected from the group consisting of a bond, -C(=O)-NH-, -NH-C(=O)-, -NH-C(=S)-NH-;
wherein the molecular weight *Mₙ* of the linear semitelechelic copolymer is in the range of from 10,000 to 100,000 g/mol, preferably in the range of from 15,000 to 40,000 g/mol (corresponding to from 100 to 280 monomer units), more preferably the molecular weight is in the range of from 20,000 to 30,000 g/mol (corresponding to from 134 to 210 monomer units);
and wherein the linear semitelechelic copolymer contains at one end of the polymer chain a functional group for attachment to a protein structure (e.g. by single-point grafting), which functional group is selected from the group comprising esters, preferably N-hydroxysuccinimide ester or C1-C4 alkyl esters, amides, preferably thiozolidine-2-thione amide, maleimide, azide, propargyl, preferably this functional group is azide or maleimide.

Further object of the present invention is a conjugation kit for labelling antibodies comprising the fluorescent polymer according to the present invention, a reaction buffer (0.1M phosphate buffer, 10 mM EDTA, pH 7), dithiothreitol (DTT) and at least one column having a volume in the range of from 0.5 to 2 mL, preferably with a volume of 0.8 mL, which contains cross-linked dextran with a molecular weight of from 100 to 5,000 g/mol, suitable for centrifugal separation of high-molecular-weight substances from low-molecular-weight substances.

The procedure for labelling antibodies using the above conjugation kit consists of the following steps:
A. Reduction of an antibody and removal of DTT from the reaction mixture.
B. Conjugation of the reduced monoclonal antibody to the fluorescent polymer.

In step A), the antibody is dissolved in the reaction buffer, and DTT in the reaction buffer is added. The reaction mixture reacts at room temperature. The product (reduced antibody in reaction buffer) is purified on a column containing cross-linked dextran having a molecular weight of from 100 to 5,000 g/mol, and added to the fluorescent polymer in the next step.

In step B), the purified reduced antibody is conjugated to the fluorescent polymer, preferably in a molar ratio of 1: 9.

Using such a conjugation kit, it is also possible to label the protein structures themselves, which have suitable functional groups (disulphide bonds) in their molecule.

Another object of the present invention is the use of the fluorescent polymer, fluorescent probe and/or conjugation kit according to the present invention for fluorescent labelling of protein structures, selected from the group consisting of peptide, protein, lipoprotein, antibody, enzyme, hormone, receptor, structural protein, transport protein, cell signalling pathway protein, synthetic protein.

The fluorescent polymer according to the present invention may carry a combination of several fluorophores bound in several different defined ratios and with an end group for single-point grafting on the protein structure. Various combinations of several fluorophores can then be used to distinguish different spectral combinations, and thus distinguish different types of cell subsets.

In one preferred embodiment, the fluorescent polymer or the fluorescent probe carries a combination of two fluorophores, one of which has fluorescence independent of the environment and thus allows to normalise the signal from different cells or environments. The other fluorophore has fluorescence dependent on various parameters of the external environment, and its fluorescence can react to changes in the external environment. Fluorescent polymers also carry an end group for single-point grafting on the protein structure single point grafting end group on the protein structure according to the present invention for use in determining various physicochemical or biological parameters of cells or environments.

Another object of the present invention is the use of the fluorescent polymer, fluorescent probe and/or conjugation kit according to the present invention in fluorescent imaging techniques, preferably selected from the group comprising flow cytometry for detecting cellular structures and markers, multiplex analysis in flow cytometry to label various antibodies against cellular structures and markers, which is compiled to detect these antibodies simultaneously on individual cells, fluorescence 'barcoding', bead assays, microscopy, western blot, fluorescence flow cytometry for the analysis of cellular events, where one of the fluorophores is responsive to this event. The second fluorophore is stable, thus setting the reference quantification level. The fluorescent polymer and/or fluorescent probe of the present invention can be used, for example, as a secondary reporter probe in sandwich ELISA assays using beads as a carrier for the detection of soluble proteins in suspension; in microscopic techniques based on fluorescence detection for imaging and detection of cellular structures and markers; in microscopic techniques based on fluorescence detection for multiplex analyses and detection of cellular structures and markers; in microscopic techniques based on fluorescence detection for analysis of cellular events, where one of the fluorophores is responsive to this event and the second fluorophore is stable, setting a reference quantification level; in western blot techniques for direct labelling and detection of proteins on the membrane; and direct multiplex labelling and detection of proteins on the membrane.

Other uses are in medical diagnostics, whole-body imaging and/or fluorescence assisted surgery, preferably in diagnosing and monitoring the success of treatment for cancers, hematopoietic diseases (leukaemia, lymphomas, hematopoietic failure), the immune system diseases (primary and secondary immunodeficiency, immune dysregulation, autoimmune diseases), both inflammatory and bacterial. The fluorescent probe and/or fluorescence kit of the present invention can be used, for example, in fluorescence detection-based whole-body imaging techniques to detect organs, single cells, cellular structures and markers; for multiplex detection of organs, single cells, cell structures and markers; for the analysis of physiological and cellular events at the level of organs, single cells, cellular organelles or components as well as soluble proteins, wherein one of the fluorophores is responsive to this event, and the other fluorophore is stable, setting the reference quantification level; in fluorescence-guided surgery to label and display fluorescence in target structures of organs and body tissues.

### Summary

Thus, the fluorescent probe and fluorescent polymer of the invention can be tailored to a given application for flow cytometry, microscopy, whole-body imaging, western blot, and other techniques using fluorescence as a reporter system. This is made possible by the choice and arbitrary combination of fluorophore and protein as a targeting and specificity-determining structure. The user can therefore create a whole spectrum of their own probes according to their requirements directly in the laboratory, for single- and multi-colour experiments, without the need to purchase different kits from different manufacturers.

The present invention will also allow the preparation of a fluorescent 'barcode' probe for multiplex analyses with the ability to distinguish 6 to 8 different fluorescent 'barcodes' in a single experiment.

The present invention further allows the determination of parameters of the outside environment or physiological parameters of cells by combining one fluorophore responsive to the respective stimulus with another fluorophore serving as an internal fluorescence standard for normalising the assay result, which represents a significant advance in fluorescence flow cytometry.

### Brief Description of the Drawings

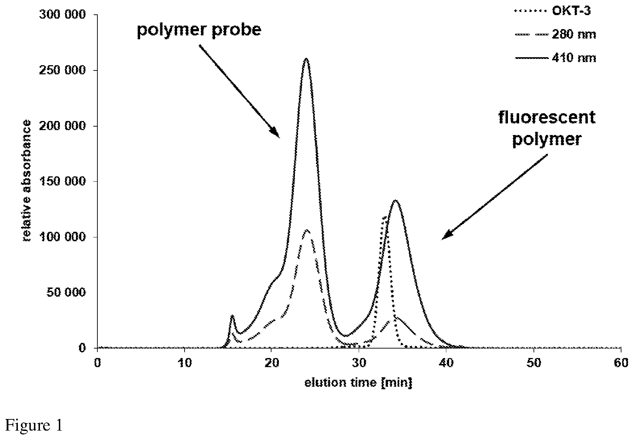

Figure 1: Compilation of GPC chromatograms of the polymer probe with the attached anti-CD3 monoclonal antibody (OKT-3) and a Dy-410-labelled fluorescent polymer (ex/em 405/460 nm), the antibody itself and the fluorescent polymer.

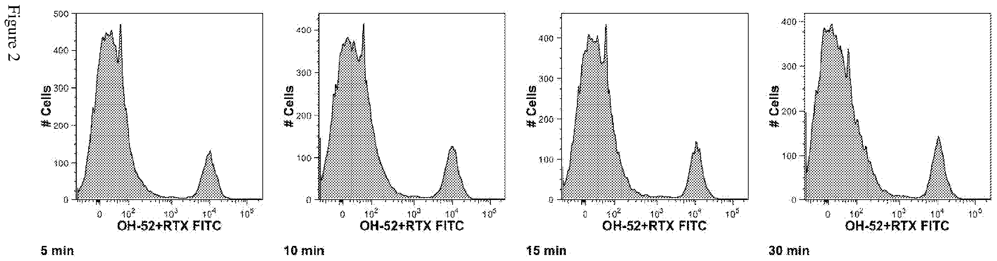

Figure 2: Result of flow cytometry of a peripheral blood sample after incubation with CD20 antibody conjugated using a rapid conjugation kit containing a fluorescent polymer with fluorescent FITC label. Histograms document an identical labelling profile over the conjugation time range of 5-30 minutes. FITC fluorescence detection is shown on the x-axis.

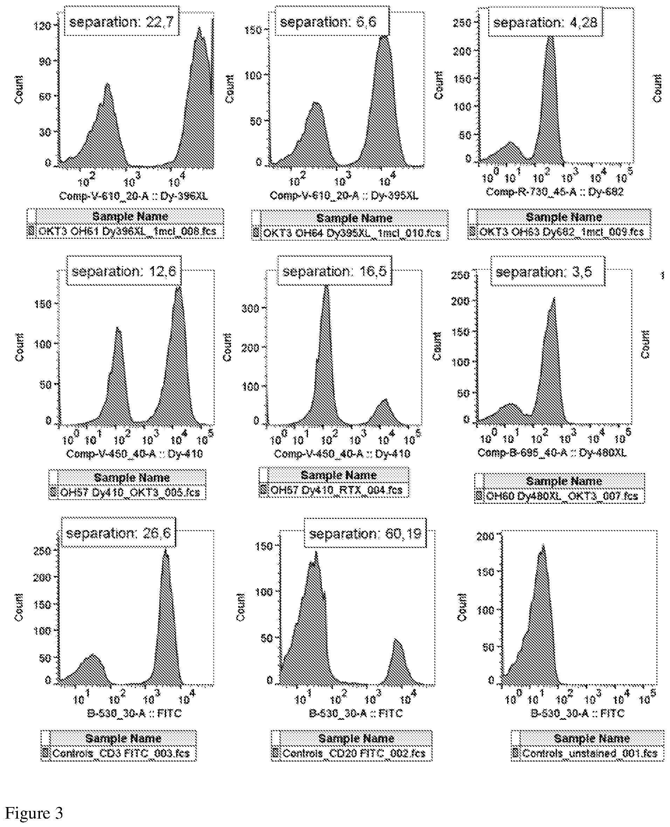

Figure 3: Result of flow cytometry on a peripheral blood sample after incubation with various polymer probes antibody-polymer-fluorophore. CD3 OKT and CD20 RTX antibodies were conjugated to the fluorescent labels indicated in the panel descriptions. The control labelling profile of commercial CD3 FITC, CD20 FITC and unlabelled lymphocytes is shown in the last line of the panels for comparison.

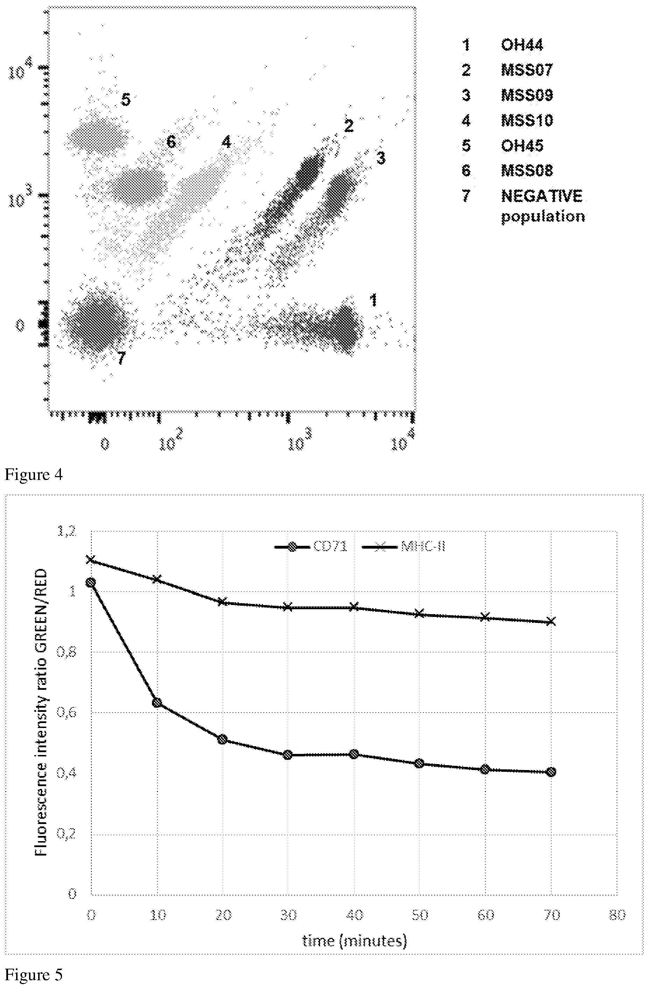

Figure 4: Fluorescence intensity of UltraComp beads (Invitrogen) dyed with antibodies conjugated to fluorescent polymers containing fluorophores Dy-490 and Dy-647P1 in various ratios (MSS-07 to MSS-10) and monochromatic fluorescent polymers with Dy-490 (OH-44) and Dy- 647P1 (OH-45). Beads without antibody binding sites (negative red population) serve as a negative control.
Figure 5: Decreasing fluorescence ratio of fluorophores GREEN/RED = FITC/DY-615 as a function of the decrease of FITC fluorescence after entry and accumulation in acidic organelles depending on the incubation time. While the rapidly internalised transferrin receptor delivered the bound anti-CD71 antibody to acidic organelles as early as the first 10 minutes of incubation, the slowly internalising MHC-II exhibits a significantly slower and weaker rate of transit to acidic organelles.

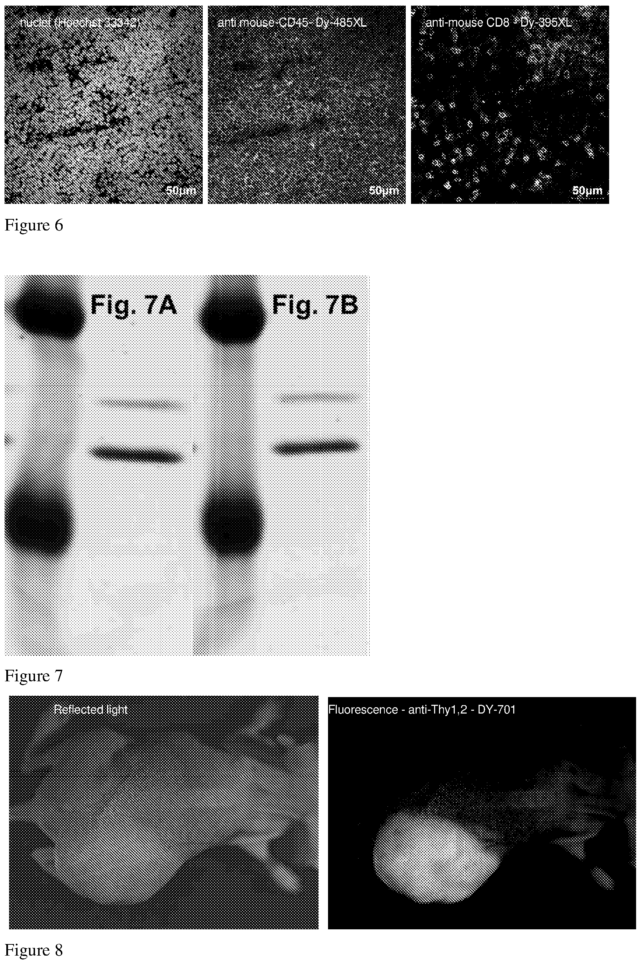

Figure 6: Tissue section from BALB/c mouse spleen labelled with a combination of fluorescent probes prepared according to Examples 4 and 6 from anti-mouse CD45 antibody (CapricoBio, USA) + semitelechelic copolymers bearing Dy-485XL and anti-mouse CD8 antibodies (CapricoBio, USA) + semitelechelic copolymers bearing Dy-395XL. Both antibodies clearly and distinctly label specific populations, CD45 all lymphocytes, and CD8 only T cells.
Figure 7: Detection of JNK kinase in cell lysate by Western blot technique using a commercial primary + secondary antibody kit (rabbit anti-JNK + anti-rabbit secondary Ab IRDye 800) Figure 7A and using a primary anti-JNK antibody directly labelled with fluorescent polymers bearing Dy-701. Both systems label the desired protein specifically and with comparable intensity.
Figure 8: Whole-body imaging of NuNu mouse bearing EL-4 tumour visualised with an anti-Thy1,2 antibody labelled according to Examples 4 and 6 with copolymer carrying fluorophore DY-701. The specific fluorescence signal of a specific fluorescence probe detects and delimits the tumour, thus enabling its precise delineation, localisation and eventual removal.

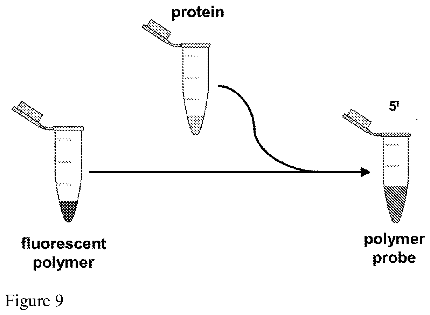

Figure 9: Schematic representation of the use of the conjugation kit.

### Examples of the invention embodiments

### Example 1: Synthesis of semitelechelic raft-poly(HPMA-co-MA-βAla-TT)-N₃ copolymer by controlled RAFT radical polymerisation

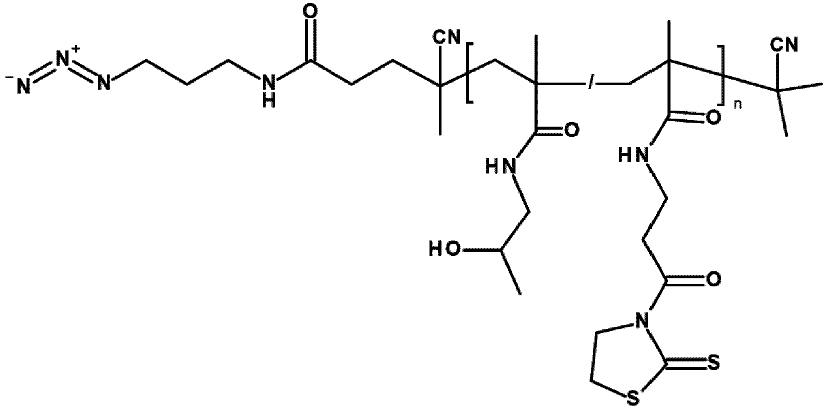

### raft-poly(HPMA-co-MA-βAla-TT)-N₃

Semitelechelic raft-poly(HPMA-co-MA-βAla-TT)-N₃ copolymer containing an end azide group (N₃) and thiazolidine-2-thione (TT) groups along the polymer chain was prepared by controlled RAFT radical polymerisation, where the molar ratio of initiator/RAFT agent/comonomers was 0.5/1/250. The ratio of HPMA/MA-βAla-TT comonomers was 90.2/9.8 mol %. 600 mg (4.19 mmol) of *N*-(2-hydroxypropyl)methacrylamide (HPMA) was dissolved in *tert*-butanol (5.530 ml), 94.13 mg (0.36 mmol) of 3-(3-methacryloylamidopropanoyl)thiazolidine-2-thione (MA-βAla-TT), 2.8 mg (9.1 µmol) of initiator, 2,2'-azobis[*N*-(2-carboxyethyl)-2-methylpropionamidine] (V-70) and 6.32 mg (18.2 µmol) of RAFT agent, *N*-(3-azidopropyl)-4-ethylsulphanyl-carbothioylsulphanyl-4-methyl-pentanamide (CTA-N₃) was dissolved in dimethylacetamide (DMA; 0.7 ml). These solutions were mixed in a polymerisation ampoule. The polymerisation mixture was bubbled with argon for 10 minutes. The copolymerisation was carried out at 40 °C for 16 hours in a sealed ampoule. The product was isolated by precipitation into a 1: 1 mixture of acetone/diethyl ether, filtered and dried to constant weight. Reactive ω-end trithiocarbonate groups (TTc) were removed with 2,2'-azobisisobutyronitrile (AIBN) to prepare a 15% solution of polymer with AIBN (20 wt %) in DMA. The solution in the ampoule was bubbled with argon for 10 minutes, sealed and left at 80 °C for 3 hours. The product was isolated by precipitation into a 1: 1 mixture of acetone/diethyl ether, filtered and dried to constant weight. Characterization of the resulting copolymer: *M*ₙ (SEC) 20,900 g/mol; *Ð* ~ 1.16; content of (TT) ~ 8.7 mol %.

Following the procedure in Example 1, other raft-poly(HPMA-co-MA-βAla-TT)-N₃ copolymers with different molar masses *M*ₙ in the range from 8,000 to 37,000 g/mol were prepared analogously, wherein the polydispersity index *Ð* was in all cases in the range of 1.01 to 1.15. The content of TT-groups along the chain was 2.2 to 9.2 mol %. According to this procedure, also other raft-poly(HPMA-co-MA-R1-TT)-N₃ copolymers were prepared, which contained in their structure aminoacyl R1 according to formula I. See Table 1.

**Table 1: Physical and chemical characterisation of semitelechelic raft-poly(HPMA-co-MA-R1-TT)-N₃ copolymers prepared according to Example 1**

| Sample | *Mₙ* (g/mol) | *Ð* | Content of TT groups (mol %) | Number of carbons in R1 |
|---|---|---|---|---|
| 1-1 | 8,000 | 1.10 | 6.5 | 3 |
| 1-2 | 11,100 | 1.09 | 2.2 | 3 |
| 1-3 | 23,300 | 1.15 | 7.5 | 3 |
| 1-4 | 27,000 | 1.05 | 9.2 | 3 |
| 1-5 | 36,700 | 1.15 | 9.0 | 3 |
| 1-6 | 27,400 | 1.01 | 7.6 | 6 |
| 1-7 | 16,500 | 1.10 | 8.3 | 6 |

### Example 2: Synthesis of semitelechelic raft-poly(HPMA-co-MA-Pr-NH-Boc)-N₃ copolymer by controlled RAFT radical polymerisation

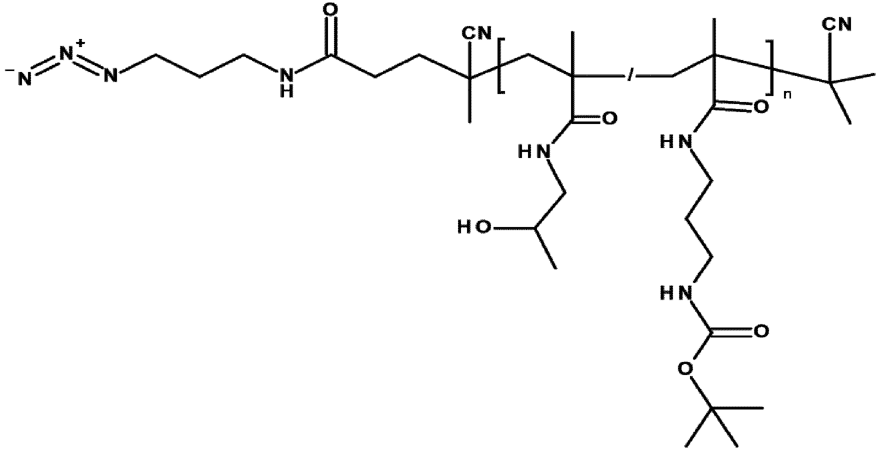

### raft-poly(HPMA-co-MA-Pr-NH-Boc)-N₃

Semitelechelic raft-poly(HPMA-co-MA-Pr-NH-Boc)-N₃ copolymer containing an end azide group (N₃) and protected amine groups (-NH-Boc) along the polymer chain was prepared by controlled RAFT radical polymerisation, where the molar ratio of initiator/RAFT agent/comonomers was 0,5/1/200. The ratio of HPMA/MA-Pr-NH-Boc comonomers was 90/10 mol %. 500 mg (3.49 mmol) of N-(2-hydroxypropyl)methacrylamide (HPMA) was dissolved in tert-butanol (4.710 ml), 89.35 mg (0.39 mmol) of methacryolyl-6-aminoropylamine protected on amine groups by tert-butyloxycarbonyl groups (MA-Pr-NH-Boc), 2.99 mg (9.7 µmol) of initiator, 2,2'-azobis[*N*-(2-carboxyethyl)-2-methylpropionamidine] (V-70) and 6.73 mg (19.4 µmol) of RAFT agent, *N*-(3-azidopropyl)-4-ethylsulphanylcarbothioylsulphanyl-4-methyl-pentanamide (CTA-N₃) was dissolve in dimethylacetamide (DMA; 0.6 ml). These solutions were mixed in a polymerisation ampoule. The polymerisation mixture was bubbled with argon for 10 minutes. The copolymerisation was carried out at 40 °C for 16 hours in a sealed ampoule. The product was isolated by precipitation into a 1: 1 mixture of acetone/diethyl ether, filtered and dried to constant weight. Reactive ω-end trithiocarbonate groups (TTc) were removed with 2,2'-azobisisobutyronitrile (AIBN) to prepare a 15% solution of polymer with AIBN (20 wt %) in DMA. The solution in the ampoule was bubbled with argon for 10 minutes, sealed and left at 80 °C for 3 hours. The product was isolated by precipitation into a 1: 1 mixture of acetone/diethyl ether, filtered and dried to constant weight. Characterization of the resulting copolymer: *M*ₙ (SEC) 23,500 g/mol; *Ð* ~ 1.07; content of (NH₂) ~ 9.7 mol %.

Following the procedure in Example 2, other raft-poly(HPMA-co-MA-Pr-NH-Boc)-N₃ copolymers with different molar masses *M*ₙ in the range from 24,500 to 44,500 g/mol were prepared analogously, wherein the polydispersity index *Ð* was in all cases in the range of 1.03 to 1.10. According to this procedure, also other raft-poly(HPMA-co-MA-R2-NH-Boc)-N₃ copolymers were prepared, which contained in their structure aminoacyl R2 according to formula II. The content of NH₂ groups along the chain was 6.7 to 8.6 mol %. See Table 2.

**Table 2: Physical and chemical characterisation of semitelechelic raft-poly(HPMA-co-MA-R2-NH-Boc)-N₃ copolymers prepared according to Example 2**

| Sample | *Mₙ* (g/mol) | *Ð* | Content of NH₂ groups (mol %) | Number of carbons in R2 |
|---|---|---|---|---|
| 2-1 | 24,700 | 1.07 | 6.7 | 3 |
| 2-2 | 27,800 | 1.03 | 7.0 | 3 |
| 2-3 | 44,400 | 1.10 | 8.6 | 3 |
| 2-4 | 25,700 | 1.05 | 8.5 | 6 |
| 2-5 | 41,300 | 1.08 | 7.8 | 6 |

### Example 3: Removal of the Boc protecting group on the amine groups of the semitelechelic raft-poly(HPMA-co-MA-Pr-NH-Boc)-N₃ copolymer

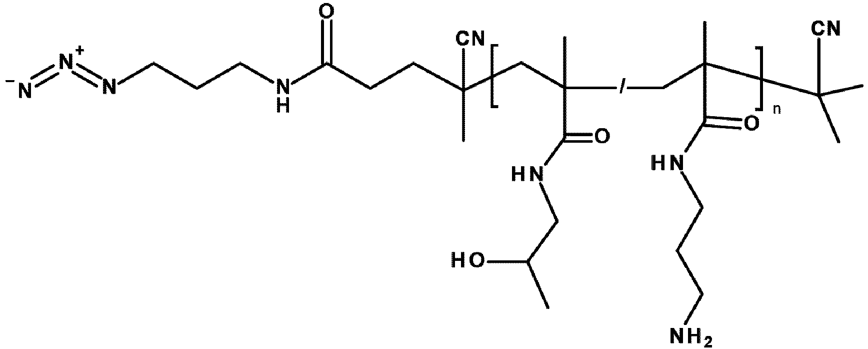

### raft-poly(HPMA-co-MA-Pr-NH₂)-N₃

The semitelechelic raft-poly(HPMA-co-MA-Pr-NH₂)-N₃ copolymer was prepared from the semitelechelic raft-poly(HPMA-co-MA-Pr-NH-Boc)-N₃ copolymer of Example 2 (348 mg), which contained amine groups protected by a *tert*-butyloxycarbonyl group (Boc). The semitelechelic raft-poly(HPMA-co-MA-Pr-NH-Boc)-N₃ copolymer was dissolved in 6.96 ml of distilled water and transferred to a polymerisation ampoule. The reaction mixture was bubbled with argon for 10 minutes. Deprotection was performed at 150 °C for 1 hour in a sealed polymerisation ampoule. The product was finally lyophilised.

Yield 297 mg (85 %). Characterization: *M*_{w} = 25,200 g/mol, *M*ₙ = 23,500 g/mol. Similarly, the protecting groups were removed from other polymers prepared according to procedure 2.

### Example 4 (reference example, not part of the invention): Preparation of fluorescentpolymer raft-poly(HPMA-co-MA-βAla-Dy-490)-N₃ by an aminolytic reaction

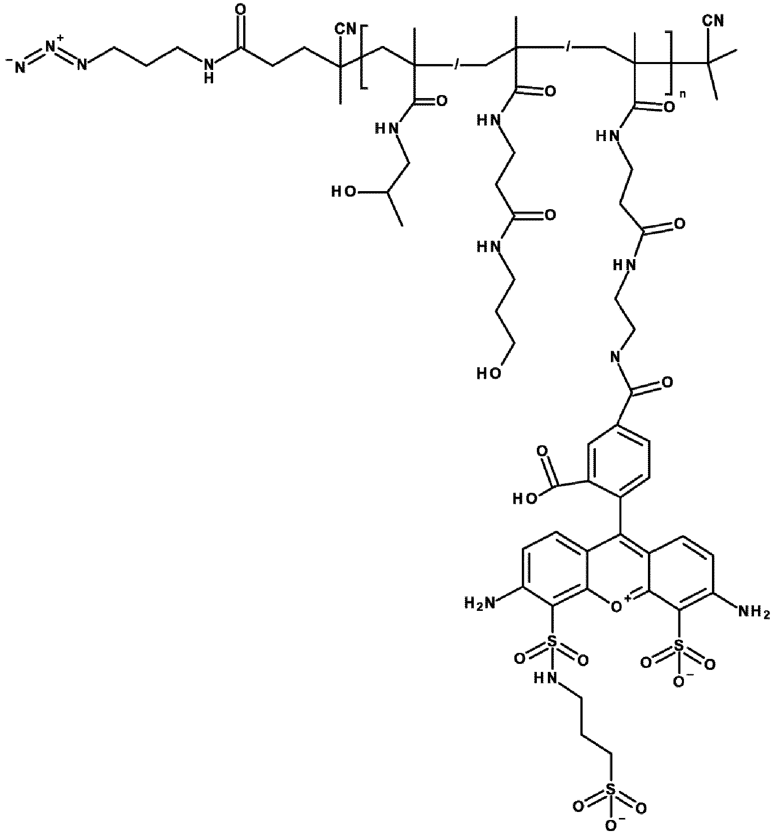

### raft-poly(HPMA-co-MA-βAla-Dy-490)-N₃

The fluorescent polymer raft-poly(HPMA-co-MA-βAla-Dy-490)-N₃ was prepared by aminolytic reaction of TT groups along the chain of the copolymer (Example 1) and the amino derivative of the fluorophore Dy-490 (ex/em 490/415 nm), which is commercially available. The starting copolymer raft-poly(HPMA-co-MA-βAla-TT)-N₃ (15 mg, 0.61 µmol) was dissolved in anhydrous dimethyl sulphoxide (DMSO, 110 µl) and a solution of the amino derivative Dy-490 (1.70 mg, 3.57 µmol, 3.5 mol %) in DMSO was added along with DIPEA (1 µl, 5.7 µmol). The reaction mixture was allowed to react in the dark for 4 hours under constant stirring. Unreacted TT groups were removed by the addition of *1*-aminopropan-2-ol (5 µl, 65 µmol). The product was purified by column chromatography (Sephadex LH20, methanol). The fraction with the sample was evaporated under vacuum, and the product was lyophilised from aqueous solution. Yield 92 % (13.7 mg). Dy-490 content ~ 3.1 mol %. Following the procedure in Example 4, other fluorescent polymers derived from the polymers prepared in Example 1 were prepared analogously with amino derivatives of various fluorophores whose excitation wavelengths were in the range of from 300 to 850 nm and emission wavelengths in the range of from 350 to 1,200 nm. The fluorophore content ranged from 0.4 to 10 mol %. See Table 3.

**Table 3: Physical and chemical characterisation of fluorescent polymers raft-poly(HPMA-co-MAR-TT)-N₃ prepared according to Example 4 (R is alkylene)**

| Sample | Fluorophore | Excitation/emission (nm) | Fluorophore content (mol %) | Number of carbons in R |
|---|---|---|---|---|
| 4-1 | Dy-396XL | 392/572 | 1.06 | 3 |
| 4-2 | Dy-395XL | 396/560 | 2.36 | 3 |
| 4-3 | Dy-410 | 405/460 | 2.82 | 3 |
| 4-4 | Dy-485XL | 485/560 | 2.04 | 6 |
| 4-5 | Dy-490 | 491/545 | 0.71 | 3 |
| 4-6 | RUB-C4 | 455/630 | 9.35 | 3 |
| 4-7 | Dy-480 | 500/630 | 1.55 | 6 |
| 4-8 | Dy-520XL | 520/664 | 0.94 | 6 |
| 4-9 | Cyanine3 | 555/570 | 1.18 | 3 |
| 4-10 | Dy-615 | 621/641 | 1.57 | 6 |
| 4-11 | Dy-647P1 | 653/672 | 0.64 | 3 |
| 4-12 | Dy-682 | 684/718 | 0.63 | 3 |
| 4-13 | Dy-701 | 706/731 | 0.52 | 3 |
| 4-14 | Sulpho-Cyanine7.5 | 778/797 | 0.40 | 6 |

### Example 5: Preparation of fluorescentpolymer raft-poly(HPMA-co-MA-Pr-NH-FITC)-N₃

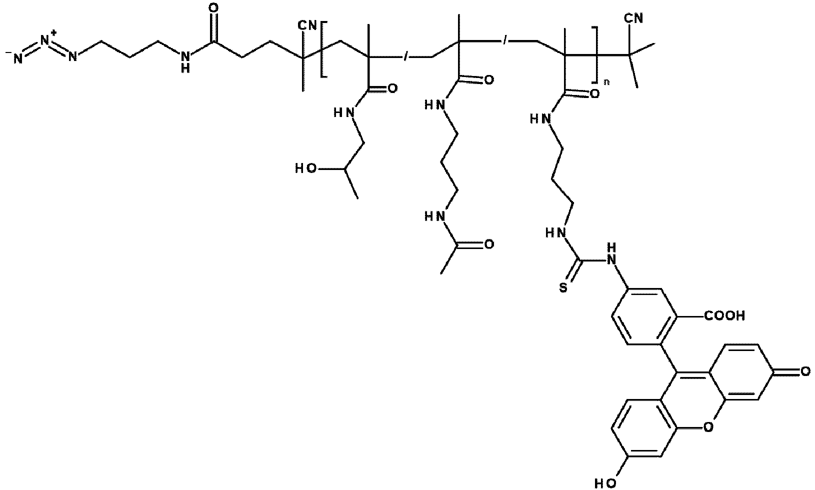

### raft-poly(HPMA-co-MA-Pr-NH-FITC)-N₃

The fluorescent polymer raft-poly(HPMA-co-MA-Pr-FITC)-N₃ was prepared by reacting NH₂ groups along the chain of a raft-poly(HPMA-co-MA-Pr-NH₂)-N₃ copolymer and fluorescein isothiocyanate (FITC, ex/em 490/520 nm). The starting copolymer (80 mg, 2.88 µmol) was dissolved in anhydrous dimethyl sulphoxide (DMSO, 570 µl) and a solution of FITC (6.52 mg, 16.8 µmol, 2.7 mol %) in DMSO was added. The reaction mixture was allowed to react in the dark for 4 hours under constant stirring. Unreacted NH₂ groups were removed by the addition of acetylthiazolidine-2-thione (1.5 mg, 15.3 µmol). The product was purified by column chromatography (Sephadex LH20, methanol). The fraction with the sample was evaporated under vacuum, and the product was lyophilised from aqueous solution. Yield 86 % (74.6 mg). FITC content ~ 2.52 mol %.

The polymer precursor raft-poly (HPMA-co-MA-Pr-NH₂)-N₃ prepared according to Example 3 was also used to prepare a fluorescent polymer using other fluorophore derivatives, e.g. N-hydroxysuccinimide esters according to the procedure described in Example 5. Other polymer precursors prepared according to Example 3 were used analogously for the preparation of fluorescent polymers.

Following the procedure in Example 5, other fluorescent polymers raft-poly(HPMA-co-MA-R-NH-FITC)-N₃ with different FITC contents ranging from 0.4 to 5 mol % were prepared analogously. See Table 4 (R is alkylene).

**Table 4 Physical and chemical characterisation of fluorescently labelled polymer precursors raft-poly(HPMA-co-MA-R-FITC)-N₃ prepared according to Example 5**

| Sample | Fluorophore | Excitation/emission (nm) | Fluorophore content (mol %) | Number of carbons in R |
|---|---|---|---|---|
| 5-1 | FITC | 490/520 | 0.43 | 3 |
| 5-2 | FITC | 490/520 | 0.86 | 3 |
| 5-3 | FITC | 490/520 | 2.18 | 6 |
| 5-4 | FITC | 490/520 | 3.34 | 6 |
| 5-5 | FITC | 490/520 | 4.71 | 3 |

### Example 6 (reference example, not part of the invention): Preparation of fluorescent polymer raft-poly(HPMA-co-MA-pAla-Dy-490)-maleimide by a click reaction

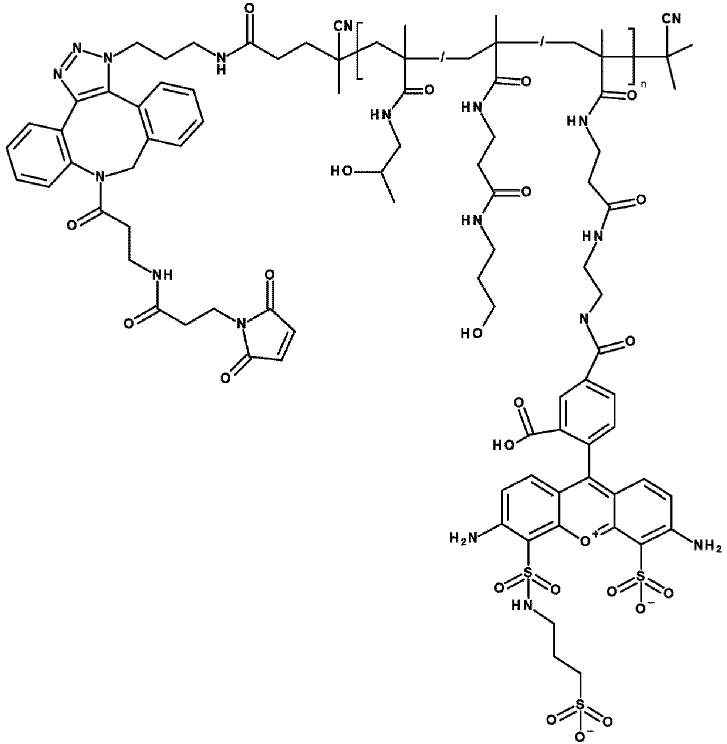

### raft-poly(HPMA-co-MA-βAla-Dy-490)-maleimide

The fluorescent polymer raft-poly(HPMA-co-MA-βAla-Dy-490)-maleimide was prepared by a click reaction of an azide group present at the end of the polymer chain of raft-poly(HPMA-coMA-βAla-Dy-490)-N₃ (Example 4) and dibenzocyclooctyne-maleimide (DBCO-MI). The starting fluorescent polymer (13.7 mg, 0.56 µmol) was dissolved in anhydrous DMSO (115 µl), and a solution of DBCO-MI (0.6 mg, 1.4 µmol) in DMSO was added. The reaction mixture was allowed to react in the dark for 2 hours under constant stirring. The formation of the product was monitored chromatographically (Shimadzu HPLC system equipped with a Chromolith Performance RP-18e reverse phase column (100 × 4.6 mm) and a Shimadzu SPD-10AVvp UV-VIS detector (310 nm); eluent 5% acetonitrile - 95% acetonitrile with gradient 0-95 % by volume 95% acetonitrile, flow rate 1 ml·min⁻¹) from the decrease of the initial amount of DBCO-MI. The product was purified by column chromatography (Sephadex LH20, methanol). The fraction with the sample was evaporated under vacuum, and the product was lyophilised from aqueous solution. Yield 93 % (12.7 mg).

In the same manner, maleimide end groups were introduced onto all fluorescent polymers prepared in Examples 4 and 5.

### Example 7 (reference example, not part of the invention): Preparation of fluorescent polymer raft-poly(HPMA-co-MA-βAla-Dy-490)-TT by a click reaction

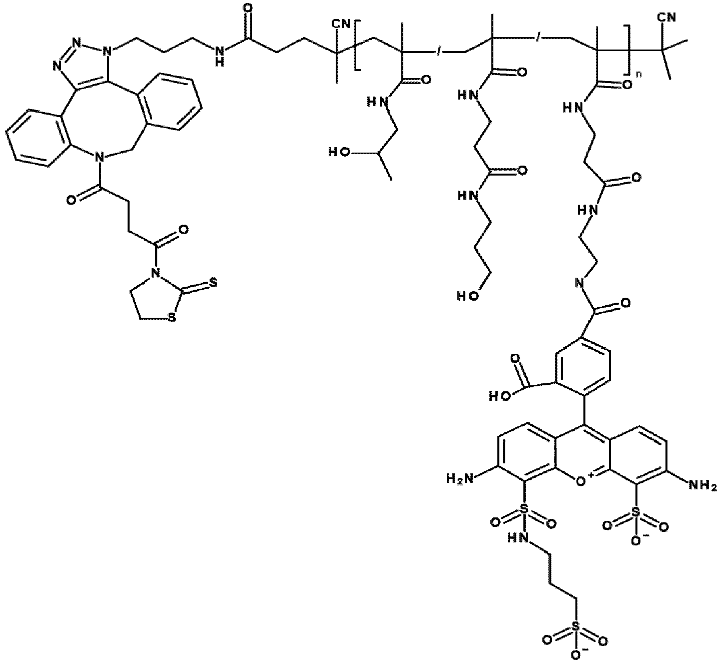

### raft-poly(HPMA-co-MA-βAla-Dy-490)-TT

The fluorescent polymer raft-poly(HPMA-co-MA-βAla-Dy-490)-TT was prepared by a click reaction of an azide group present at the end of the polymer chain of raft-poly(HPMA-co-MA-βAla-Dy-490)-N₃ (Example 4) and dibenzocyclooctyne-carboxylic acid (DBCO-Cbx), which was subsequently modified with thiazolidine-2-thione. The starting fluorescent polymer (15.0 mg, 0.61 µmol) was dissolved in anhydrous DMSO (120 µl), and a solution of DBCO-Cbx (0.47 mg, 1.52 µmol) in DMSO was added. The reaction mixture was allowed to react in the dark for 2 hours under constant stirring. The formation of the product was monitored chromatographically (Shimadzu HPLC system equipped with a Chromolith Performance RP-18e reverse phase column (100 × 4.6 mm) and a Shimadzu SPD-10AVvp UV-VIS detector (310 nm); eluent 5% acetonitrile - 95% acetonitrile with a gradient of 0-95% by volume 95% acetonitrile, flow rate 1 ml·min⁻¹) from the decrease of the starting amount of DBCO-Cbx. Subsequently, N-(3-dimethylaminopropyl)-N'-ethylcarbodiimide hydrochloride (44 mg, 2.31 µmol), thiazolidine-2-thione (0.22 mg, 1.93 µmol) and a catalytic amount of dimethyl aminopyridine were added to the reaction mixture. The reaction mixture was allowed to react in the dark for 2 hours under constant stirring. The product was purified by column chromatography (Sephadex LH20, methanol). The fraction with the sample was evaporated under vacuum, and the product was precipitated as described in Examples 1 and 2. The TT content was determined spectrophotometrically. Yield 88 % (13.2 mg), TT content 0.92 % mol. In the same manner, TT end groups were introduced on all fluorescent polymers prepared in Examples 4 and 5.

### Example 8 (reference example, not part of the invention): Preparation of fluorescent polymer raft-poly(HPMA-co-MA-βAla-Dy-490)-N-hydroxysuccinimide ester by a click reaction

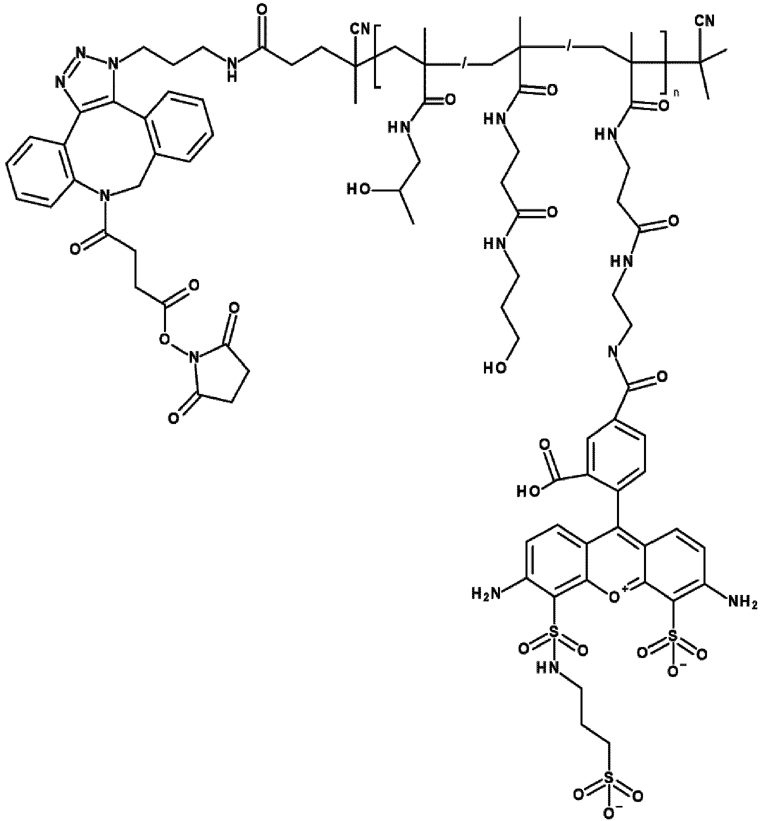

### raft-poly(HPMA-co-MA-βAla-Dy-490)-NHS

The fluorescent polymer raft-poly(HPMA-co-MA-βAla-Dy-490)-*N*- hydroxysuccinimide ester was prepared by a click reaction of an azide group present at the end of the polymer chain of raft-poly(HPMA-co-MA-βAla-Dy-490)-N₃ (Example 4) and dibenzocyclooctyne-N-hydroxysuccinimide ester (DBCO-NHS). The starting fluorescent polymer (12.0 mg, 0.49 µmol) was dissolved in anhydrous DMSO (90 µl), and a solution of DBCO-NHS (0.49 mg, 1.22 µmol) in DMSO was added. The reaction mixture was allowed to react in the dark for 2 hours under constant stirring. The formation of the product was monitored chromatographically (Shimadzu HPLC system equipped with a Chromolith Performance RP-18e reverse phase column (100× 4.6 mm) and a Shimadzu SPD-10AVvp UV-VIS detector (310 nm); eluent 5% acetonitrile - 95% acetonitrile with a gradient of 0-95% by volume 95% acetonitrile, flow rate 1 ml·min⁻¹) from the decrease of the starting amount of DBCO-NHS. The product was purified by column chromatography (Sephadex LH20, methanol). The fraction with the sample was evaporated under vacuum, and the product was precipitated as described in Examples 1 and 2. In the same manner, NHS end groups were introduced on all fluorescent polymers prepared in Examples 4 and 5.

### Example 9: Preparation of a fluorescent probe with bound anti-CD20 monoclonal antibody by an aminolytic reaction

A fluorescent probe **PS1** with bound anti-CD20 monoclonal antibody was prepared by aminolytic reaction of the end TT groups present on the fluorescent polymers (Example 7) and the amino groups present on the antibody molecule. A solution of anti-CD20 monoclonal antibody was added to the fluorescent polymer in a molar ratio of anti-CD20: fluorescent polymer = 1:20. The reaction mixture was allowed to react in the dark for 5 minutes under constant stirring; the product was desalted on a PD10 column (Sephadex G25) in water and lyophilised. The presence of the resulting fluorescent probe was confirmed by size exclusion chromatography (SEC) on a Superose6 column in 0.3M acetate buffer with pH 6.5. Following the same procedure, a fluorescent probe **PS2** was prepared with a bound monoclonal antibody and a fluorescent polymer containing end *N-*hydroxysuccinimide ester groups (Example 8). Similarly, fluorescent probes were prepared from other monoclonal antibodies.

### Example 10: Reduction of disulphide bonds present in an anti-CD3 monoclonal antibody molecule by dithiothreitol (DTT)

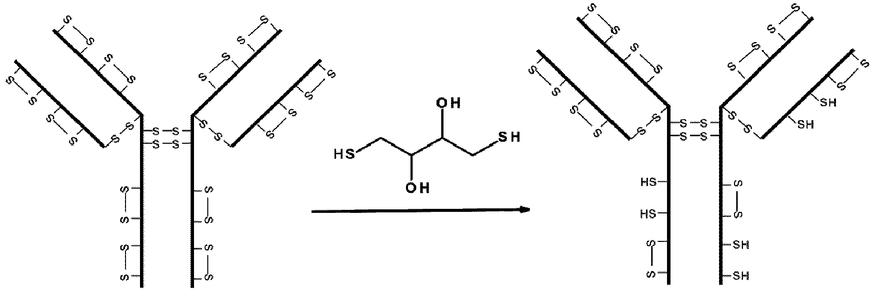

The reduction of disulphide bonds was carried out in the presence of dithiothreitol in reaction phosphate buffer (0.1M phosphate buffer, 10 mM EDTA; pH 7), which was bubbled with argon for 30 minutes before use. To a solution of anti-CD3 monoclonal antibody (0.028 µmol; OKT-3), dithiothreitol (1.54 mmol; DTT) was added, and the mixture was allowed to react for 5 minutes under constant stirring. The product was purified on a PD10 column (Sephadex G25) in reaction buffer. The anti-CD3 monoclonal antibody thus prepared was immediately conjugated to the fluorescent polymer prepared according to Example 6. Characterisation: sulphhydryl group content ~ 9.8.

Following the procedure in Example 10, other reduced monoclonal antibodies were prepared analogously, such as anti-CD-4, anti-CD-20, anti-CD-8, anti-CD45, anti-Thy1,2, anti-MHC II, anti JNK.

### Example 11: Preparation of a polymer probe with the bound anti-CD3 monoclonal antibody by a click reaction

A fluorescent probe **PS3** with bound anti-CD3 monoclonal antibody was prepared by a click reaction of the end maleimide groups present on the fluorescent polymers (Example 6) and the sulphhydryl groups present on the reduced antibody (Example 10). A solution of purified reduced anti-CD3 monoclonal antibody was added to the fluorescent polymer in a molar ratio of anti-CD3 : fluorescent polymer = 1: 9. The reaction mixture was allowed to react in the dark for 5 minutes under constant stirring; the product was desalted on a PD10 column (Sephadex G25) in water and lyophilised. The presence of the resulting fluorescent probe was confirmed by size exclusion chromatography (SEC) on a Superose6 column in 0.3M acetate buffer with pH 6.5. The GPC chromatogram of the fluorescent probe is shown in Figure 1. Fluorescent probes **PS4** to **PS6** were prepared from other monoclonal antibodies according to the same procedure.

### Example 12: Modification of sulphhydryl groups present in the anti-CD3 monoclonal antibody molecule by a click reaction

DBCO groups for binding azide-terminated fluorescent polymers (Examples 4 and 5) were introduced onto the sulphhydryl groups present on the reduced antibody (Example 10) using sulphodibenzocyclooctyne-maleimide (sDBCO-MI). sDBCO-MI binding was carried out in reaction phosphate buffer (0.1M phosphate buffer, 10 mM EDTA; pH 7), which was bubbled with argon for 30 minutes before use. sDBCO-MI (0.69 µmol) was added to a solution of anti-CD3 monoclonal antibody (0.034 µmol; OKT-3), and the mixture was allowed to react for 60 minutes under constant stirring. The product was purified on a PD10 column (Sephadex G25) in distilled water and lyophilised. The modified anti-CD3 monoclonal antibody thus prepared was used for conjugation on a fluorescent polymer according to Example 14.

Following this procedure, other monoclonal antibodies with modified sulphhydryl groups were prepared analogously.

### Example 13: Modification of amino groups present in the anti-CD3 monoclonal antibody molecule by a click reaction

The amino groups of the lysine residues present on the antibody were modified to bind the azide-terminated fluorescent polymers (Examples 4 and 5) using dibenzocyclooctyne-N-hydroxysuccinimide ester (DBCO-NHS). DBCO-NHS binding was carried out in reaction phosphate buffer (0.1M phosphate buffer, 10 mM EDTA; pH 7). A solution of DBCO-NHS (6.80 µmol) in DMSO was added to the solution of anti-CD3 monoclonal antibody (0.034 µmol; OKT-3), and the mixture was allowed to react for 60 minutes under constant stirring. The product was purified on a PD10 column (Sephadex G25) in distilled water and lyophilised. The modified anti-CD3 monoclonal antibody thus prepared was used for conjugation to a fluorescent polymer prepared according to Examples 4 and 5.

Following this procedure, other monoclonal antibodies with modified amino groups were prepared analogously.

### Example 14: Preparation of a fluorescent probe with bound anti-CD3 monoclonal antibody by a click reaction

Fluorescent probes **PS7** and **PS8** with bound anti-CD3 monoclonal antibody were prepared by a click reaction of the end azide groups present on the fluorescent polymers (Examples 4 and 5) and the DBCO groups introduced on the antibody molecules (Examples 12 and 13). A solution of anti-CD3 monoclonal antibody in a buffer (0.1M phosphate buffer, 10 mM EDTA, pH 7) was added to the fluorescent polymer in a molar ratio of anti-CD3: fluorescent polymer = 1:20. The reaction mixture was allowed to react in the dark for 5 minutes under constant stirring; the product was desalted on a PD10 column (Sephadex G25) in water and lyophilised. The presence of the resulting fluorescent probe was confirmed by size exclusion chromatography (SEC) on a Superose6 column in 0.3M acetate buffer with pH 6.5. Fluorescent probes with other antibodies and fluorophores were prepared according to the same procedure.

**Table 5 Overview and characterisation of prepared types of fluorescent probes**

| Fluorescent probe | End group on the polymer | Antibody | Reactive groups on the antibody | Number of polymer chains on the antibody | Number of fluoropho res on PS | Apparent molecular weight⁴ |
|---|---|---|---|---|---|---|
| PS1 | TT | anti-CD3 | Lys-NH₂ | 4.3 | 18.5 | 255,000 |
| PS2 | NHS | anti-CD3 | Lys-NH₂ | 3.9 | 20.3 | 245,000 |
| PS3 | MI | anti-CD3 | Cys-SH¹ | 6.2 | 26.0 | 303,000 |
| PS4 | MI | anti-CD4 | Cys-SH¹ | 7.2 | 33.8 | 329,000 |
| PS5 | MI | anti-CD8 | Cys-SH¹ | 8.1 | 43.7 | 352,000 |
| PS6 | MI | anti-CD20 | Cys-SH¹ | 6.5 | 28.6 | 311,000 |
| PS7 | N₃ | anti-CD3 | -DBCO² | 5.7 | 26.2 | 290,000 |
| PS8 | N₃ | anti-CD3 | -DBCO³ | 5.4 | 24.8 | 283,000 |

| | | | | | | |
|---|---|---|---|---|---|---|
| ¹ SH groups were obtained by reduction of disulphide bonds. ² DBCO groups were introduced onto the antibody to SH groups using sDBCO-maleimide. ³ DBCO groups were introduced onto the antibody on NH₂ groups using DBCO-NHS. ⁴ The apparent molar mass of the polymer probe was determined as the sum of the molecular weight of the antibody and *Mₙ* of the polymer chains bound to the antibody. | | | | | | |

### Example 15: Preparation of a fluorescent polymer for the preparation of a multi-spectral fluorescent nanoprobe

A fluorescent polymer for the preparation of a multi-spectral fluorescent nanoprobe was prepared by aminolytic reaction of the fluorophores Dy-490 and Dy-647P1 with the appropriate polymer precursor. The appropriate Dy-490 and Dy-647P1 derivatives were added to the reaction mixture in a 1:1 molar ratio sequentially after 5 minutes. The following procedure was the same as in Examples 4, 5 and 6. Characterization: fluorophore content: Dy-409 ~ 0.45 mol %; Dy-647P1 ~ 0.40 mol %. The molar ratio of fluorophores: 1.15: 1.

Following the procedure in Example 15, other fluorescent polymers were prepared analogously to prepare multi-spectral fluorescent nanoprobes with different fluorophores and their mutual molar ratios. Their excitation wavelengths were in the range of from 300 to 850 nm and emission wavelengths in the range of from 350 to 1,200 nm. The content of fluorophores ranged from 0.2 to 3 mol %. See Table 6. Other fluorescent polymers containing fluorophore pairs were prepared in the same manner.

**Table 6: Physical and chemical characterisation of fluorescent polymers for the preparation of a multi-spectral fluorescent probe prepared according to Example 15**

| Sample | Fluorophore 1 (FL1) | | | Fluorophore 2 (FL2) | | | FL1: FL2 ratio |
|---|---|---|---|---|---|---|---|
| | Name | Ex/Em | Content (mol %) | Name | Ex/Em | Content (mol %) | |
| 15-1 | Dy-490 | 490/515 | 0.55 | Dy-647P1 | 650/670 | 1.49 | 1:2.7 |
| 15-2 | Dy-490 | 490/515 | 1.45 | Dy-647P1 | 650/670 | 0.28 | 5.2:1 |
| 15-3 | Dy-490 | 490/515 | 0.20 | Dy-647P1 | 650/670 | 2.06 | 1:10.8 |
| 15-4 | Dy-490 | 490/515 | 2.76 | Dy-647P1 | 650/670 | 0.48 | 1:5.8 |
| 15-5 | Dy-396XL | 396/576 | 0.38 | Dy-647P1 | 650/670 | 0.43 | 1:1.1 |
| 15-6 | Dy-396XL | 396/576 | 0.54 | Dy-647P1 | 650/670 | 1.66 | 1:3.1 |
| 15-7 | Dy-396XL | 396/576 | 1.56 | Dy-647P1 | 650/670 | 0.47 | 3.3:1 |

### Example 16: Preparation of a multi-spectral fluorescent nanoprobe using a fluorescent polymer and a reduced anti-CD3 monoclonal antibody

A multi-spectral fluorescent nanoprobe was prepared by conjugating a fluorescent polymer (Example 15) and a reduced anti-CD3 monoclonal antibody (Example 10) according to the procedure described in Example 11.

### Example 17: Example of characterisation of fluorescentpolymers and fluorescent probes

The prepared copolymers, fluorescent polymers and fluorescent probes with monoclonal antibodies were characterised by determining the weight and number average of molecular weights (*M_{w}, Mₙ*) and the corresponding polydispersity index (*Ð*) using size exclusion chromatography (SEC) on a system equipped with a UV detector (Shimadzu, Japan), RI detector (Optilab REX, Wyatt Technology Corp., USA) and a multi-angle light scattering detector (DAWN Heleos-II, Wyatt Technology Corp., USA). In the case of SEC, a TSK 3000 Super SW column was used for characterisation and a mixture of MeOH (80 %) and 0.3M acetate buffer with pH 6.5 (20 %) as the mobile phase; a Superose6 column was used for characterisation of monoclonal antibody probes with 0.3M acetate buffer with pH 6.5 as the mobile phase. The sample concentration was 3 mg/ml in all cases.

The content of TT and NH₂ groups, copolymerised statistically along the polymer chain, was determined spectrophotometrically. All measurements were made on a Specord 205 UV-VIS spectrophotometer (Analytik Jena, Germany). The content of TT groups was determined according to the literature (Šubr, V.; Koňák, Č.; Laga, R.; Ulbrich, K. Biomacromolecules 2006, 7(1): 122-130) in methanol (*e₃₀₅ =* 10,800 l·mol⁻¹· cm⁻¹). The content of amine groups (after deprotection in distilled water at 150 °C in a sealed polymerisation ampoule) was determined according to the method described in Fields R. Methods Enzymol. 1972; 25: 464-468 (*e₄₂₀ =* 11,550 l·mol⁻¹·cm⁻¹). The content of bound fluorophores was determined spectrophotometrically.

### Example 18: Effect of reaction time on the fluorescence probe function

The fluorescent polymer (polymer precursor with FITC fluorescent label according to Examples 5 and 6) was used for conjugation to CD20-RTX monoclonal antibody for 5 minutes (A), 10 minutes (B), 15 minutes (C) and 30 minutes (D). The fluorescent polymer with FITC and the antibody were incubated for 15 minutes with a human peripheral blood sample by a conventional procedure (incubation with the antibody, lysis of erythrocytes with ammonium chloride solution and centrifugation). The prepared cell suspension was measured with a flow cytometer (BD Celesta, 488 nm laser excitation and 530/30 nm emission detection). All times tested resulted in equally significant fluorescence intensity of CD20-RTX FITC (Fig. 2), which was comparable to the commercially available CD20 FITC reagents.

As expected, the blood sample contains a mixture of T-lymphocytes, NK cells and B-lymphocytes, with only B-lymphocytes carrying a CD20 molecule on the surface, which is detected by a polymer probe and represented by a positive peak in the right part of the graph. The negative signal on the left of the figure belongs to T-lymphocytes and NK cells that do not carry the CD20 marker. **Thus, the fluorescent probe provides the expected result with an extremely short conjugation time. The resulting polymeric fluorescent probe is useful for detecting human cells carrying an antibody-targeted molecule.**

### Example 19: Example of the use of a conjugation kit for labelling antibodies

The conjugation kit for labelling antibodies consists of fluorescent polymers which carry various fluorescent labels along the chain (Examples 4 and 5) and end maleimide groups. It also contains reaction buffer (0.1M phosphate buffer, 10 mM EDTA, pH 7), dithiothreitol (DTT) and columns containing cross-linked dextran with a molecular weight of 100 to 5,000 g/mol (Sephadex G-25) with a volume of 0.8 ml (PD SpinTrap G-25), which is suitable for centrifugal separation of high-molecular-weight substances from low-molecular-weight impurities. The procedure consists of two necessary steps:
A. Reduction of monoclonal antibody and removal of DTT from the reaction mixture. 150 µg of monoclonal antibody was dissolved in 100 µl of reaction buffer, and 231 µg of DTT in 50 µl of reaction buffer was added. The reaction mixture was allowed to react under the conditions described in Example 10. The product (150 µg of reduced monoclonal antibody in 150 µl of reaction buffer) was purified using a PD SpinTrap G-25 column and added to the fluorescent polymer in the next step.
B. Conjugation of a reduced monoclonal antibody to a fluorescent polymer. The reduced monoclonal antibody was conjugated to the fluorescent polymer in a molar ratio of 1: 9 (150 µg of reduced monoclonal antibody and 260 µg of the fluorescent polymer at *Mₙ* = 27,800 g/mol) according to the procedure of Example 11. The mixture was allowed to react for 5 minutes, and subsequently, the resulting product was used for cytometric analysis.

Using such a conjugation kit, it is also possible to label the protein structures themselves, which have suitable functional groups in their molecule (see Fig. 9).

### Example 20: Variable design of polymerfluorescent probes

Several conjugation kits have been prepared, which consist of a fluorescent polymer with a fluorescent label (fluorophores) with different excitation and emission characteristics (Dy-396XL; Dy395XL; s Dy682; Dy410; Dy480XL). We prepared polymer fluorescent probes by rapid conjugation with CD3 OKT3 or CD20 RTX antibody. These probes were incubated for 15 minutes with human peripheral blood samples according to standard procedures. The prepared cell suspension was measured with a BD Celesta flow cytometer, with excitation at 488 nm, 405 nm and/or 640 nm, and emission detection in the bands indicated in the x-axis label in Figure 3. All prepared and tested polymer fluorescent probes led to the expected profile of labelling of the lymphocyte sample, CD3 on T-lymphocytes and CD20 on B-lymphocytes. The separation of positive and negative cells was variable according to the selected fluorophore and the efficiency of its excitation by available lasers. **Polymer fluorescent probes allow the detection of cells carrying the target molecule. The variability of the conjugation kit then provides flexibility in the selection of the antibody according to the user's needs, these polymer fluorescent probes can be detected in different channels of the flow cytometer, and thus the optimal composition of antibody conjugates can be tailored to the research or diagnostic task.**

### Example 21: Multi-spectral conjugation kits

Conjugation kits with a multi-colour spectral profile were prepared. The fluorescent polymer was prepared by binding a mixture of two fluorophores Dy-490 and Dy-647P1 in different ratios according to Example 15. At the same time, fluorescent polymers with individual fluorophores Dy-490 and Dy-647P1 were prepared. Fluorescent polymers were conjugated to the CD8 OKT-8 antibody. We tested the principle of the possibility of distinguishing particles labelled with individual types of polymers with different spectrally distinguishable characteristics using UltraComp particles (Invitrogen) stained with antibodies conjugated to polymers containing fluorophores Dy-490 and Dy-647P1 in various ratios and monochromatic polymers with Dy-490 and Dy-647P1. The particles were incubated in six separate aliquots with each type of fluorescent polymer and antibody separately. We performed the measurements using a flow cytometer. As an unlabelled control sample, we added UltraComp particles without incubation with the polymer fluorescent probe. As documented in Figure 4, the different types of spectrally unique polymers can be distinguished as separate groups of particles with the same characteristics (distinguished in Fig. 4). In this way, different types of particles can be labelled (the so-called barcoding) for efficient analysis of sample mixtures using multi-coloured antibody panels. By combining only two fluorophores, it makes it possible to distinguish six types of barcode. The successful use of **multi-spectral fluorescent probes to distinguish six original samples in just two flow cytometer detection channels has been documented.**

### Example 22: Physiological probes

Anti-CD71 and anti-MHCII antibodies were labelled with a combination of fluorescent polymers carrying a combination of two fluorophores (Examples 4, 5 and 6). One fluorophore is FITC, in which the fluorescence decreases in a lower pH environment, and the other fluorophore is Dyomics-615, which exhibits stable pH-independent fluorescence over the pH range of 3.5 to 9. Isolated mouse splenocytes (BALB/c strain) were homogenised and washed in HBSS. They were then cooled and labelled with antibodies at 4 °C for 20 minutes. Subsequently, they were washed, transferred to a culture medium at 37 °C and analysed at a 10-minute interval on a BD LSR-II flow cytometer. As a parameter, the emission ratio of FITC vs Dy-615 was analysed over time.

Figure 5 shows a significant change in the fluorophore emission ratio with increasing incubation time. As the antibody enters the cell, it is internalised into acidic organelles (lysosomes, late endosomes), where the quantum yield of FITC emission decreases while Dy-615 emission remains at baseline. The rapidly internalising transferrin receptor (CD71) shows a rapid change in the fluorescence ratio, while the slowly internalising MHC-II shows a continuous slow decline.

In this way, it is possible to very accurately monitor the rate of internalisation of surface structures depending on the antibody used and to calculate and analyse the kinetics of absorption and transition to acidic intracellular compartments, either by flow cytometry or by quantitative imaging techniques.

### Example 23: Microscopy

The anti-mouse CD45 antibody (CapricoBio, USA) was labelled with a fluorescent polymer carrying Dy-485XL (Examples 4 and 6), and the anti-mouse CD8 antibody (CapricoBio, USA) was labelled with a fluorescent polymer carrying Dy-395XL (Examples 4 and 6). Spleen tissues were sectioned from sacrificed BALB/c mice, cut into small pieces, placed in OCT freezing medium (Sakura Finetek, Torrance, CA) and stored at -80 °C. Tissue sections (8 µm thick) were fixed in chilled acetone for 5 minutes, air dried, washed with cold PBS and blocked with 10% normal horse serum (Jackson ImmunoResearch, West Grove, PA) for 30 minutes at room temperature (RT). Labelling with antibodies was carried out at 4 °C for 20 minutes with a prepared mixture of both antibodies simultaneously. After washing with cold PBS, they were fixed with 1% paraformaldehyde and transferred to an aqueous medium containing DAPI (Sigma) and visualised on an Olympus FV-1000 microscope at the appropriate wavelengths (Dy-485XL Ex 480 nm and Em 570/40 nm, Dy-395XL Ex 405 nm and Em 570/40 nm).

Figure 6 clearly shows green labelled lymphocyte populations (anti-CD45) and red labelled T-cells (anti-CD8), the nuclei of all cells in the preparation are labelled blue with DAPI.

Both user-labelled antibodies can be thus successfully used in combination for multiplex analysis of preparations for fluorescence microscopy.

### Example 24: Western Blot

The anti-JNK antibody was labelled with a conjugation kit that uses fluorescent polymers carrying the fluorophore Dy-701 (Examples 4 and 6).

Cell lysates were separated on 12% SDS-PAGE, transferred onto nitrocellulose membranes and blocked in 5% skimmed milk in PBS-T. The membrane was incubated with an unlabelled rabbit anti-JNK antibody or with an anti-JNK antibody labelled with a fluorescent polymer carrying the fluorophore Dy-701. The secondary antibody used to visualise the membrane labelled with an unlabelled antibody was IRDye 800 (anti-rabbit). Detection and quantification were carried out using an Odyssey infrared imaging system (LI-Cor Biosystems).

Figure 7 shows that the antibody labelled directly with the conjugation kit (Fig. 7B) achieves identical results as the use of the secondary professionally labelled antibody (Fig. 7A).

### Example 25: In vivo imaging

The anti-mouse Thy 1.2 antibody was labelled with the conjugation kit using a fluorescent polymer carrying the fluorophore Dy-701 (Examples 4 and 6). A total of 1·10⁶ mouse thymoma EL-4 cells were injected subcutaneously into the right flank of NuNu mice, and the tumour growth was noted.

*In vivo* imaging was carried out on an OV100 Whole Mouse System (Olympus Corp.) containing an MT-20 light source and an Orca II ERG CCD camera (Hamamatsu, Japan). Animals were anaesthetised by intraperitoneal injection of 1% Narkamon in the amount of 300 µl per mouse. Imaging of tumour growth was non-invasive; the accumulation of specific antibody in the tumour mass was observed through the skin covering the tumour. The fluorescence of the antibody specifically labelling the tumour cells was visualised using specific filters (Ex 700 nm, Em 750/40). Images were taken separately for the fluorescence channel and the reflected light, then combined and stained in AnalySIS 3.2.822 software (SIS Systems).

Figure 8 clearly shows the accumulation of fluorescence in the tumour mass detectable as early as 12 hours after administration of the labelled fluorescent probe. The localisation of the fluorescent signal up to the periphery of the tumour then clearly allows the tumour to be localised and its exact boundaries defined.

## Claims

1. A fluorescent polymer, comprising a linear semitelechelic statistical copolymer to which at least one fluorescent label is bound in an amount in the range of from 0.4 to 12 mol %, based on the number of monomer units,
wherein the linear semitelechelic statistical polymer comprises polyacrylamide, polymethacrylamide, or poly(*N*-(2-hydroxypropyl)methacrylamide, wherein from 0.4 to 12 mol % of monomer units in the linear semitelechelic statistical copolymer are replaced by monomer units of general formula (I) 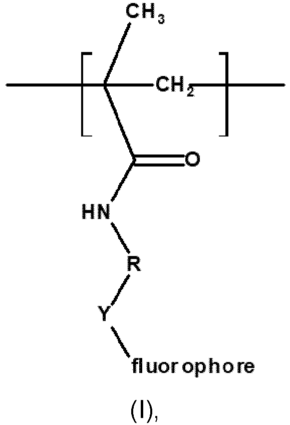
wherein
R is selected from the group consisting of a straight or branched carbon alkylenyl chain with a number of carbons from 1 to 6; wherein R may be further substituted with one or more identical or different natural amino acid side chains;
Y is selected from the group consisting of a bond, -C(=O)-NH-, -NH-C(=O)-, -NH-C(=S)-NH-; wherein the molecular weight Mₙ of the linear semitelechelic copolymer is in the range of from 10,000 to 100,000 g/mol;
and wherein the fluorophore has a molecular weight in the range of from 350 to 1,500 g/mol, an excitation wavelength in the range of from 300 to 850 nm and an emission wavelength in the range of from 350 to 1,200 nm, and is covalently bound to the monomer unit of general formula (I) of the linear semitelechelic copolymer via terminal amine or thiocyanate or N-hydroxysuccinimide ester, wherein the groups formed following the fluorophore binding are subsequently part of the group Y;
and wherein the fluorescent polymer contains at one end of the polymer chain a functional group for binding to a protein structure, which functional group is selected from the group comprising esters, preferably N-hydroxysuccinimide ester or (C1-C4)alkyl esters, amides, preferably thiozolidine-2-thione amide, maleimide, azide, propargyl, preferably the functional group is azide or maleimide.

2. The fluorescent polymer according to claim 1, wherein the linear semitelechelic statistical copolymer comprises poly(*N*-(2-hydroxypropyl)methacrylamide, wherein from 0.4 to 12 mol % of monomer units are replaced by monomer units of general formula (I).

3. The fluorescent polymer according to any one of the preceding claims 1 or 2, which further comprises up to 11.6 mol % of monomer units of general formula (II), based on the number of monomer units, 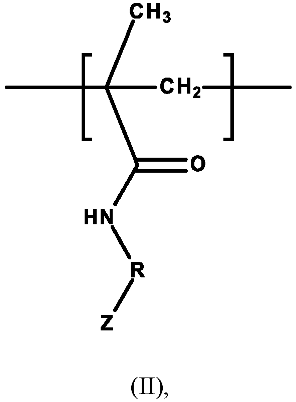 wherein
R is as defined in claim 1,
and Z is selected from the group comprising -C(=O)-NH-(CH₂)ₐ-CH₂(OH); -C(=O)-NH-(CH₂)_{b}-CH(OH)-CH₃; -C(=O)-NH-(CH₂)_{b}-CH(OH)-(CH₂)_{c}-CH₃; wherein *a* is an integer from 0 to 4, *b* is an integer from 0 to 3 and *c* is from 1 to 4; -NH-C(=O)-CH₃;
wherein total content of the monomer units of general formula (I) and (II) in the fluorescent polymer is at most 12 mol %, based on the number of monomer units.

4. The fluorescent polymer according to any one of the preceding claims 1 to 3, wherein the fluorophore is selected from the group comprising fluorescein, fluorescein isothiocyanate, derivatives of ruthenium-bipyridine complexes Ru(bpy)₃, in particular bis(2,2'-bipyridine)-[4-(4'-methyl-2,2'-bipyridin-4-yl)butan-1-aminium ruthenium tris(perchlorate), cyanines, 3,6-diamino-9-[4-(2-aminoethylcarbamoyl)-2-carboxyfenyl]-5-(3 -sulphonatopropylsulphamoyl)xanthene-10-ium-4-sulphonate, (2E)-1-[6-(2,5-dioxopyrro-lidin-1-yl)oxy-6-oxohexyl]-2-[(2E,4E)-5-[1-(2-methoxyethyl)-3,3-dimethyl-5-sulphonato-indol-1-ium-2-yl]penta-2,4-dienylidene]-3,3-dimethyl-indoline-5-sulphonate, 3-[4-[(E)-2-[6-[6-(2-aminoethylamino)-6-oxohexoxy]-1,3-benzoxazol-2-yl]vinyl]-1-pyridyl]propane-1-sulphonate, 3-[4-[6-[[6-(2,5-dioxopyrrolidin-1-yl)oxy-6-oxohexyl]carbamoylamino]-1,3-benzoxazol-2-yl]pyridin-1-ium-1-yl]propane-1-sulphonate, 3-[[6-(2-azaniumyl-ethylamino)-6-oxohexyl]carbamoyl]-8-chloro-7-hydroxy-2-oxo-chromene-6-sulphonate, 3-[4-[7-[[6-(2-aminoethylamino)-6-oxohexyl]-ethyl-amino]-2-oxo-chromen-3-yl]pyridin-1-ium-1-yl]propane-1-sulphonate, 1-[6-(2-aminoethylamino)-6-oxohexyl]-6-[(E)-2-[7-(diethylamino)-2-oxo-chromen-3-yl]vinyl]pyridin-1-ium-3-sulphonate, 1-[6-(2-aminoethylamino)-6-oxo-hexyl]-4-[(E)-2-[7-(diethylamino)-2-oxo-chromen-3-yl]vinyl]pyridin-1-ium-3-sulphonate, (2E)-2-[(E)-3-(7-amino-2-*terc*-butyl-chromenylium-4-yl) prop-2-enylidene]-1-[6-(2-aminoethylamino)-6-oxohexyl]-3,3-dimethyl-indoline-5-sulphonate, (2Z)-3-[4-(2-aminoethylamino)-4-oxo-butyl]-2-[(E)-3-[4-*terc*-butyl-7-[ethyl(3-sulpho-natopropyl)amino]chromenylium-2-yl]prop-2-enylidene]-3-methyl-1-(3-sulphonatopropyl)indo-line-5-sulphonate, and (2Z)-3-[4-(2-aminoethylamino)-4-oxo-butyl]-2-[(E)-3-[7-(diethylamino)-3-methyl-4-phenyl-chromenylium-2-yl]prop-2-enylidene]-3-methyl-1-(3-sulpho-natopropyl)indoline-5-sulphonate, or optionally their derivatives containing a functional group selected from an amino group, an isothiocyanate group and an N-hydroxysuccinimide group.

5. The fluorescent polymer according to any one of the preceding claims 1 to 4, which comprises at least two different fluorophores.

6. A fluorescent probe comprising at least one fluorescent polymer according to any one of the claims 1 to 5, to which a protein structure is covalently bound via an end group of the linear polymeric chain;
wherein the protein structure is selected from the group consisting of a peptide having from 10 to 200 amino acids, a protein, a lipoprotein, an antibody, an enzyme, a hormone, or a cellular receptor; preferably the protein structure is a monoclonal antibody, and the fluorescent polymer is covalently bound to a light chain of the monoclonal antibody, more preferably to a light chain of the monoclonal antibody, selected from the group comprising anti-CD20, anti-CD3, anti-CD4, anti-CD8, anti-CD71, anti-MHCII, anti-CD45, anti-JNK.

7. A method of preparing the fluorescent probe according to claim 6, **characterized in that** it comprises the following steps:
a) Providing the monomers of the linear semitelechelic copolymer, selected from the group comprising N-(2-hydroxypropyl)methacrylamide, acrylamide, methacrylamide;
and monomers of the general formula (IV) 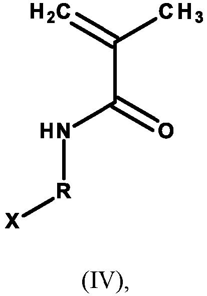
wherein
R is selected from the group consisting of a straight or branched carbon alkylene chain with a number of carbons from 1 to 6; wherein R may be further substituted with one or more of the same or different natural amino acid side chains;
and
X is a carbonyl-thiazoline-2-thione group or an -NH₂ group, wherein the -NH₂ group may optionally be protected with a protecting group;
b) Polymerisation of the monomers resulting in formation of the linear semitelechelic copolymer;
c) Binding a fluorophore to the linear semitelechelic copolymer to form a fluorescent polymer;
d) Optionally, modification of a protein structure;
e) Binding of the protein structure to the fluorescent polymer.

8. The method of preparing the fluorescent probe according to claim 7, **characterized in that** step b) of polymerisation of the monomers is carried out by controlled RAFT radical polymerisation of the monomers from step a) with a content of 0.4 to 12 mol % of the monomer of general formula (IV), and at least 88 mol % of monomer units selected from the group comprising *N*-(2-hydroxypropyl)methacrylamide, acrylamide, methacrylamide;
wherein the polymerisation is carried out at a temperature in the range of from 30 to 100 °C, and a solvent preferably selected from the group comprising dimethylsulphoxide, dimethylacetamide, dimethylformamide, methanol, ethanol, dioxane, tetrahydrofuran, propanol, tert-butanol or mixtures thereof;
the reaction is initiated by an initiator, preferably selected from the group comprising 2,2'-azobis[N-(2-carboxyethyl)-2-methylpropionamidine] and *N*-(3-azidopropyl)-4-[(Z)-[4-(3-azidopropylamino)-1-cyano-1-methyl-4-oxobutyl] azo]-4-cyano-pentanamide;
optionally in the presence of a transfer agent, preferably *N*-(3-azidopropyl)-4-ethylsulphanylcarbothioylsulphanyl-*4*-methyl-pentanamide;
optionally followed by removal of protecting groups protecting the amine groups of the side chains.

9. The method of preparing the fluorescent probe according to claim 7 or 8, **characterized in that** step c) of binding a fluorophore to the linear semitelechelic copolymer is performed by conjugating the free amine groups or carbonyl-thiazolidine-2-thione groups of the monomer units of general formula (IV) of the linear semitelechelic copolymer to a fluorophore, which contains a reactive amine or SCN group,
wherein the fluorophore has a molecular weight in the range of from 350 to 1,500 g/mol, the excitation wavelengths in the range of from 300 to 850 nm, and the emission wavelengths in the range of from 350 to 1,200 nm;
and wherein the fluorophore is bound to the linear semitelechelic copolymer by an amide or thioamide bond;
optionally any unreacted thiazolidine-2-thione groups are removed by reaction with amino alcohol, selected from the group comprising NH₂-(CH₂)ₐ-CH₂(OH); NH₂-(CH₂)_{b}-CH(OH)-CH₃; NH₂-(CH₂)_{b}-CH(OH)-(CH₂)_{c}-CH₃; wherein *a* is an integer from 0 to 4, *b* is an integer from 0 to 3 and c is from 1 to 4, and/or the unreacted -NH₂ groups are removed by reaction with acetylthiazolidine-2-thione;
optionally, the resulting fluorescent polymer is subjected to further reactions leading to a change of the reactive group at the end of the semitelechelic chain, preferably to a click reaction of an azide group with dibenzocyclooctyne maleimide to form a maleimide functional group at one end of the linear polymer chain;
or to a click reaction of an azide group with dibenzocyclooctyne-carboxylic acid and subsequently with thiazolidine-2-thione to form a fluorescent polymer with a thiazolidine-2-thione functional group at one end of the linear polymer chain;
or to a click reaction of an azide group with a dibenzocyclooctyne-N-hydroxysuccinimide ester to form a fluorescent polymer with an N-hydroxysuccinimide ester functional group at one end of the linear polymer chain.

10. The method of preparing the fluorescent probe according to any one of claims 7, 8 or 9, **characterized in that** step d) of the modification of a protein structure is carried out by reducing the disulphide bonds of the protein structure with a reducing agent, selected from the group of dithiothreitol, tris(2-carboxyethyl)phosphine, *β*-mercaptoethanol, glutathione; in an aqueous buffer at pH from 6.5 to 7.5 and at room temperature, to form -SH groups in the protein structure, which may optionally be further modified by reaction with sulphodibenzocyclooctyne maleimide to form sulphodibenzocyclooctyne groups;
and/or free NH₂ groups of the protein structure are modified to dibenzocyclooctyne groups by a reaction with dibenzocyclooctyne-*N*-hydroxysuccinimide ester.

11. The method of preparing the fluorescent probe according to any one of claims 7, 8, 9 or 10, **characterized in that** the step e) of binding the protein structure to the fluorescent polymer is carried out by conjugating of at least one fluorescent polymer according to any one of claims 1 to 5; and the protein structure, optionally the protein structure with introduced sulphhydryl, sulphodibenzocyclooctyne or dibenzocyclooctyne groups prepared in step d),
wherein the reaction takes place at room temperature in aqueous buffer and pH from 6.5 to 7.5.

12. A conjugation kit for labelling antibodies, **characterised in that** it comprises the fluorescent polymer according to any one of claims 1 to 5; a reaction buffer of pH 7, comprising 0.1M phosphate buffer and 10 mM EDTA; dithiothreitol; and at least one column having a volume in the range of from 0.5 to 2 mL, which contains cross-linked dextran with a molecular weight of from 100 to 5,000 g/mol.

13. Use of the fluorescent polymer according to any one of claims 1 to 5 and/or of the conjugation kit according to claim 12, for fluorescent labelling of protein structures, selected from the group consisting of peptide, protein, lipoprotein, antibody, enzyme, hormone, receptor, structural protein, transport protein, cell signalling pathway protein, synthetic protein.

14. Use of the fluorescent polymer according to any one of claims 1 to 5 and/or of the fluorescent probe according to claim 6, in fluorescent imaging techniques, preferably selected from the group comprising flow cytometry, bead assays, microscopy, western blot, fluorescence flow cytometry.

15. The fluorescent probe according to claim 6 for use as a contrast agent in medical diagnostics, whole-body imaging and/or fluorescence assisted surgery, preferably in diagnostics of cancer, inflammatory and bacterial diseases.

## Patentansprüche

1. Ein fluoreszierendes Polymer, umfassend ein lineares semitelecheles statistisches Copolymer, an das mindestens ein fluoreszierender Marker in einer Menge im Bereich von 0,4 bis 12 Mol-%, bezogen auf die Anzahl der Monomereinheiten, gebunden ist,
wobei das lineare semitelechele statistische Polymer Polyacrylamid, Polymethacrylamid oder Poly(N-(2-hydroxypropyl)methacrylamid) umfasst, wobei 0,4 bis 12 Mol-% der Monomereinheiten in dem linearen semitelechelen statistischen Copolymer durch Monomereinheiten der allgemeinen Formel (I) ersetzt sind 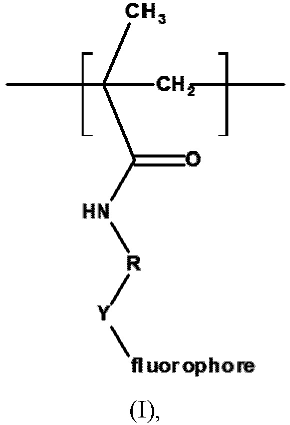
wobei
R ausgewählt ist aus der Gruppe bestehend aus einer geraden oder verzweigten Kohlenstoffalkylenylkette mit einer Anzahl von Kohlenstoffen von 1 bis 6; wobei R zusätzlich durch eine oder mehrere identische oder unterschiedliche natürliche Aminosäureseitenketten substituiert sein kann;
Y ausgewählt ist aus der Gruppe bestehend aus einer Bindung, -C(=O)-NH-, -NH-C(=O)-, -NH-C(=S)-NH-; wobei das Molekulargewicht Mₙ des linearen semitelechelen Copolymers im Bereich von 10.000 bis 100.000 g/mol liegt;
und wobei der Fluorophor ein Molekulargewicht im Bereich von 350 bis 1.500 g/mol, eine Excitationswellenlänge im Bereich von 300 bis 850 nm und eine Emissionswellenlänge im Bereich von 350 bis 1.200 nm aufweist und über terminales Amin oder Thiocyanat oder *N-*Hydroxysuccinimidester kovalent an die Monomereinheit der allgemeinen Formel (I) des linearen semitelechelen Copolymers gebunden ist, wobei die nach der Fluorophorbindung gebildeten Gruppen anschließend Teil der Gruppe Y sind;
und wobei das fluoreszierende Polymer an einem Ende der Polymerkette eine funktionelle Gruppe zur Bindung an eine Proteinstruktur enthält, welche funktionelle Gruppe ausgewählt ist aus der Gruppe umfassend Ester, vorzugsweise N-Hydroxysuccinimidester oder (C1-C4)Alkylester, Amide, vorzugsweise Thiozolidin-2-thionamid, Maleimid, Azid, Propargyl, vorzugsweise ist die funktionelle Gruppe Azid oder Maleimid.

2. Das fluoreszierende Polymer nach Anspruch 1, wobei das lineare semitelechele statistische Copolymer Poly(N-(2-hydroxypropyl)methacrylamid) umfasst, wobei 0,4 bis 12 Mol-% der Monomereinheiten durch Monomereinheiten der allgemeinen Formel (I) ersetzt sind.

3. Das fluoreszierende Polymer nach einem der vorhergehenden Ansprüche 1 oder 2, das zusätzlich bis zu 11,6 Mol-% Monomereinheiten der allgemeinen Formel (II), bezogen auf die Anzahl der Monomereinheiten, umfasst, 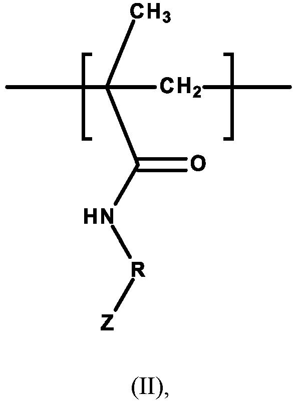 wobei
R wie in Anspruch 1 definiert ist,
und Z ausgewählt ist aus der Gruppe umfassend -C(=O)-NH-(CH₂)ₐ-CH₂(OH); -C(=O)-NH-(CH₂)_{b}-CH(OH)-CH₃; -C(=O)-NH-(CH₂)₆-CH(OH)-(CH₂)_{c}-CH₃; wobei *a* eine Ganzzahl von 0 bis 4 ist, b eine Ganzzahl von 0 bis 3 ist und c von 1 bis 4; -NH-C(=O)-CH₃;
wobei der Gesamtgehalt der Monomereinheiten der allgemeinen Formel (I) und (II) im fluoreszierenden Polymer höchstens 12 Mol-% beträgt, bezogen auf die Anzahl der Monomereinheiten.

4. Das fluoreszierende Polymer nach einem der vorhergehenden Ansprüche 1 bis 3, wobei der Fluorophor ausgewählt ist aus der Gruppe umfassend Fluorescein, Fluoresceinisothiocyanat, Derivate von Ruthenium-Bipyridin-Komplexen Ru(bpy)₃, insbesondere Bis(2,2'-bipyridin)-[4-(4'-methyl-2,2'-bipyridin-4-yl)butan-1-aminiumrutheniumtris(perchlorat), Cyanine, 3,6-Diamino-9-[4-(2-aminoethylcarbamoyl)-2-carboxyfenyl]-5-(3-sulfonatopropylsulfamoyl)xanthen-10-ium-4-sulfonat, (2E)-1-[6-(2,5-Dioxopyrrolidin-1-yl)oxy-6-oxohexyl]-2-[(2E,4E)-5-[1-(2-methoxyethyl)-3,3-dimethyl-5-sulfonato-indol-1-ium-2-yl]penta-2,4-dienyliden]-3,3-dimethyl-indolin-5-sulfonat, 3-[4-[(E)-2-[6-[6-(2-aminoethylamino)-6-oxohexoxy]-1,3-benzoxazol-2-yl]vinyl]-1-pyridyl]propan-1-sulfonat, 3-[4-[6-[[6-(2,5-Dioxopyrrolidin-1-yl)oxy-6-oxohexyl]carbamoylamino]-1,3-benzoxazol-2-yl]pyridin-1-ium-1-yl]propan-1-sulfonat, 3-[[6-(2-Azaniumyl-ethylamino)-6-oxohexyl]carbamoyl]-8-chlor-7-hydroxy-2-oxo-chromen-6-sulfonat, 3-[4-[7-[[6-(2-Aminoethylamino)-6-oxohexyl]-ethyl-amino]-2-oxo-chromen-3-yl]pyridin-1-ium-1-yl]propan-1-sulfonat, 1-[6-(2-Aminoethylamino)-6-oxo-hexyl]-6-[(E)-2-[7-(diethylamino)-2-oxo-chromen-3-yl]vinyl]pyridin-1-ium-3-sulfonat, 1-[6-(2-Aminoethylamino)-6-oxo-hexyl]-4-[(E)-2-[7-(diethylamino)-2-oxo-chromen-3-yl]vinyl]pyridin-1-ium-3-sulfonat, (2E)-2-[(E)-3-(7-Amino-2-terc-butyl-chromenylium-4-yl)-prop-2-enyliden]-1-[6-(2-aminoethylamino)-6-oxohexyl]-3,3-dimethyl-indolin-5-sulfonat, (2Z)-3-[4-(2-Aminoethylamino)-4-oxo-butyl]-2-[(E)-3-[4-tert-butyl-7-[ethyl(3-sulfonatopropyl)amino]chromenylium-2-yl]prop-2-enylidene]-3-methyl-1-(3-sulfonatopropyl)indo-line-5-sulfonate und (2Z)-3-[4-(2-Aminoethylamino)-4-oxo-butyl]-2-[(E)-3-[7-(diethylamino)-3-methyl-4-phenyl-chromenylium-2-yl]prop-2-enylidene]-3-methyl-1-(3-sulfonatopropyl)indoline-5-sulfonate oder gegebenenfalls deren Derivate, die eine funktionelle Gruppe enthalten, die aus einer Aminogruppe, einer Isothiocyanatgruppe und einer N-Hydroxysuccinimidgruppe ausgewählt ist.

5. Das fluoreszierende Polymer nach einem der vorhergehenden Ansprüche 1 bis 4, das mindestens zwei verschiedene Fluorophore umfasst.

6. Eine Fluoreszenzsonde, die mindestens ein fluoreszierendes Polymer nach einem der Ansprüche 1 bis 5 umfasst, an das eine Proteinstruktur über eine Endgruppe der linearen Polymerkette kovalent gebunden ist;
wobei die Proteinstruktur ausgewählt ist aus der Gruppe bestehend aus einem Peptid mit 10 bis 200 Aminosäuren, einem Protein, einem Lipoprotein, einem Antikörper, einem Enzym, einem Hormon oder einem zellulären Rezeptor; vorzugsweise ist die Proteinstruktur ein monoklonaler Antikörper, und das fluoreszierende Polymer ist kovalent an eine leichte Kette des monoklonalen Antikörpers gebunden, bevorzugter an eine leichte Kette des monoklonalen Antikörpers, ausgewählt aus der Gruppe bestehend aus Anti-CD20, Anti-CD3, Anti-CD4, Anti-CD8, Anti-CD71, Anti-MHCII, Anti-CD45, Anti-JNK.

7. Verfahren zur Herstellung der Fluoreszenzsonde nach Anspruch 6, **dadurch gekennzeichnet, dass** es die folgenden Schritte umfasst:
a) Bereitstellen der Monomere des linearen semitelechelen Copolymers, ausgewählt aus der Gruppe, die N-(2-Hydroxypropyl)methacrylamid, Acrylamid, Methacrylamid umfasst;
und Monomere der allgemeinen Formel (IV) 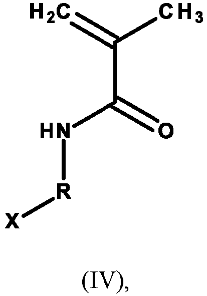
wobei
R ausgewählt ist aus der Gruppe, die aus einer geraden oder verzweigten Kohlenstoffalkylenkette mit einer Anzahl von Kohlenstoffen von 1 bis 6 besteht; wobei R zusätzlich durch eine oder mehrere der gleichen oder unterschiedlichen natürlichen Aminosäureseitenketten substituiert sein kann;
und
X ist eine Carbonylthiazolin-2-thion-Gruppe oder eine -NH₂-Gruppe, wobei die -NH₂-Gruppe optional mit einer Schutzgruppe geschützt sein kann;
b) Polymerisation der Monomere, was zur Bildung des linearen semitelechelen Copolymers führt;
c) Binden eines Fluorophors an das lineare semitelechele Copolymer, um ein fluoreszierendes Polymer zu bilden;
d) optionale Modifikation einer Proteinstruktur;
e) Bindung der Proteinstruktur an das fluoreszierende Polymer.

8. Verfahren zur Herstellung der Fluoreszenzsonde nach Anspruch 7, **dadurch gekennzeichnet, dass** Schritt b) der Polymerisation der Monomere durch kontrollierte RAFT-Radikalpolymerisation der Monomere aus Schritt a) mit einem Gehalt von 0,4 bis 12 Mol-% des Monomers der allgemeinen Formel (IV) und mindestens 88 Mol-% Monomereinheiten ausgewählt aus der Gruppe umfassend N-(2-Hydroxypropyl)methacrylamid, Acrylamid, Methacrylamid durchgeführt wird;
wobei die Polymerisation bei einer Temperatur im Bereich von 30 bis 100 °C und einem Lösungsmittel durchgeführt wird, das vorzugsweise ausgewählt ist aus der Gruppe umfassend Dimethylsulfoxid, Dimethylacetamid, Dimethylformamid, Methanol, Ethanol, Dioxan, Tetrahydrofuran, Propanol, tert-Butanol oder Mischungen davon;
die Reaktion wird durch einen Initiator eingeleitet, der vorzugsweise aus der Gruppe ausgewählt ist, die 2,2'-Azobis[N-(2-carboxyethyl)-2-methylpropionamidin] und N-(3-Azidopropyl)-4-[(Z)-[4-(3-azidopropylamino)-1-cyano-1-methyl-4-oxobutyl] azo]-4-cyano-pentanamid umfasst;
optional in Gegenwart eines Übertragungsmittels, vorzugsweise N-(3-Azidopropyl)-4-ethylsulfanylcarbothioylsulfanyl-4-methyl-pentanamid;
optional gefolgt von der Entfernung von Schutzgruppen, die die Amingruppen der Seitenketten schützen.

9. Verfahren zur Herstellung der Fluoreszenzsonde nach Anspruch 7 oder 8, **dadurch gekennzeichnet, dass** Schritt c) des Bindens eines Fluorophors an das lineare semitelechele Copolymer durch Konjugieren der freien Amingruppen oder Carbonylthiazolidin-2-thiongruppen der Monomereinheiten der allgemeinen Formel (IV) des linearen semitelechelen Copolymers mit einem Fluorophor, der eine reaktive Amin- oder SCN-Gruppe enthält, durchgeführt wird;
wobei der Fluorophor ein Molekulargewicht im Bereich von 350 bis 1.500 g/mol, die Excitationswellenlängen im Bereich von 300 bis 850 nm und die Emissionswellenlängen im Bereich von 350 bis 1.200 nm aufweist;
und wobei der Fluorophor durch eine Amid- oder Thioamidbindung an das lineare semitelechele Copolymer gebunden ist;
optional werden alle nicht umgesetzten Thiazolidin-2-thion-Gruppen entfernt durch Reaktion mit Aminoalkohol, ausgewählt aus der Gruppe bestehend aus NH₂-(CH₂)ₐ-CH₂(OH); NH₂-(CH₂)₆-CH(OH)-CH₃; NH₂-(CH₂)_{b}-CH(OH)-(CH₂)_{c}-CH₃, wobei *a* eine ganze Zahl von 0 bis 4, *b* eine ganze Zahl von 0 bis 3 und *c* von 1 bis 4 ist, und/oder die nicht umgesetzten -NH₂-Gruppen werden durch Reaktion mit Acetylthiazolidin-2-thion entfernt;
optional wird das resultierende fluoreszierende Polymer weiteren Reaktionen unterzogen, die zu einer Änderung der reaktiven Gruppe am Ende der semitelechelen Kette führen, vorzugsweise einer Klickreaktion einer Azidgruppe mit Dibenzocyclooctinmaleimid, um eine Maleimid-Funktionsgruppe an einem Ende der linearen Polymerkette zu bilden;
oder einer Klickreaktion einer Azidgruppe mit Dibenzocyclooctincarbonsäure und anschließend mit Thiazolidin-2-thion zur Bildung eines fluoreszierenden Polymers mit einer Thiazolidin-2-thion-Funktionsgruppe an einem Ende der linearen Polymerkette;
oder einer Klickreaktion einer Azidgruppe mit einem Dibenzocyclooctin-N-Hydroxysuccinimidester zur Bildung eines fluoreszierenden Polymers mit einer N-Hydroxysuccinimidester-Funktionsgruppe an einem Ende der linearen Polymerkette.

10. Verfahren zur Herstellung der Fluoreszenzsonde nach einem der Ansprüche 7, 8 oder 9, **dadurch gekennzeichnet, dass** Schritt d) der Modifikation einer Proteinstruktur wird durch die Reduktion von Disulfidbindungen der Proteinstruktur mit einem Reduktionsmittel durchgeführt, das aus der Gruppe von Dithiothreitol, Tris(2-carboxyethyl)phosphin, β-Mercaptoethanol, Glutathion ausgewählt ist; in einem wässrigen Puffer bei einem pH-Wert von 6,5 bis 7,5 und bei Raumtemperatur, um -SH-Gruppen in der Proteinstruktur zu bilden, die optional durch Reaktion mit Sulfodibenzocyclooctinmaleimid weiter modifiziert werden können, um Sulfodibenzocyclooctingruppen zu bilden;
und/oder freie NH₂-Gruppen der Proteinstruktur werden durch eine Reaktion mit Dibenzocyclooctin-N-hydroxysuccinimidester zu Dibenzocyclooctingruppen modifiziert.

11. Verfahren zur Herstellung der Fluoreszenzsonde nach einem der Ansprüche 7, 8, 9 oder 10, **dadurch gekennzeichnet, dass** der Schritt e) des Bindens der Proteinstruktur an das Fluoreszenzpolymer durch Konjugieren von mindestens einem Fluoreszenzpolymer nach einem der Ansprüche 1 bis 5 und die Proteinstruktur, optional die Proteinstruktur mit eingeführten Sulfhydryl-, Sulfodibenzocyclooctin- oder Dibenzocyclooctingruppen, die in Schritt d) hergestellt wurde, durchgeführt wird;
wobei die Reaktion bei Raumtemperatur in wässrigem Puffer und einem pH-Wert von 6,5 bis 7,5 stattfindet.

12. Ein Konjugationskit zum Markieren von Antikörpern, **dadurch gekennzeichnet, dass** es das fluoreszierende Polymer nach einem der Ansprüche 1 bis 5; einen Reaktionspuffer mit einem pH-Wert von 7, der 0,1 M Phosphatpuffer und 10 mM EDTA; Dithiothreitol; und mindestens eine Säule mit einem Volumen im Bereich von 0,5 bis 2 ml, die vernetztes Dextran mit einem Molekulargewicht von 100 bis 5.000 g/mol enthält, umfasst.

13. Verwendung des fluoreszierenden Polymers nach einem der Ansprüche 1 bis 5 und/oder des Konjugationskits nach Anspruch 12 zur Fluoreszenzmarkierung von Proteinstrukturen, ausgewählt aus der Gruppe bestehend aus Peptid, Protein, Lipoprotein, Antikörper, Enzym, Hormon, Rezeptor, Strukturprotein, Transportprotein, Zellsignalwegprotein, synthetischem Protein.

14. Verwendung des fluoreszierenden Polymers nach einem der Ansprüche 1 bis 5 und/oder der Fluoreszenzsonde nach Anspruch 6 in Fluoreszenzbildgebungsverfahren, vorzugsweise ausgewählt aus der Gruppe bestehend aus Durchflusszytometrie, Bead-Assays, Mikroskopie, Western Blot, Fluoreszenz-Durchflusszytometrie.

15. Die Fluoreszenzsonde nach Anspruch 6 zur Verwendung als Kontrastmittel in der medizinischen Diagnostik, Ganzkörperbildgebung und/oder fluoreszenzunterstützten Chirurgie, vorzugsweise in der Diagnostik von Krebs, entzündlichen und bakteriellen Erkrankungen.

## Revendications

1. Un polymère fluorescent, comprenant un copolymère statistique semi-téléchélique linéaire auquel au moins une sonde fluorescente est liée en une quantité comprise entre 0,4 et 12 % en moles, par rapport au nombre d'unités monomères,
dans lequel le polymère statistique semi-téléchélique linéaire comprend du polyacrylamide, du polyméthacrylamide ou du poly(N-(2-hydroxypropyl)méthacrylamide, dans lequel de 0,4 à 12 % en moles d'unités monomères dans le copolymère statistique semi-téléchélique linéaire sont remplacées par des unités monomères de formule générale (I) 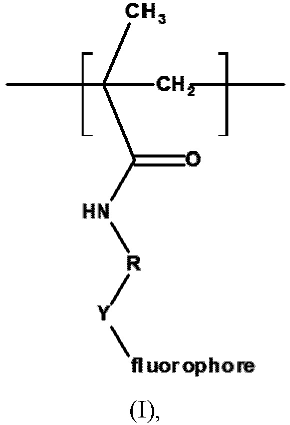
dans laquelle
R est choisi dans le groupe constitué d'une chaîne alkylényle carbonée linéaire ou ramifiée avec un nombre de carbones de 1 à 6 ; dans laquelle R peut être en outre substitué par une ou plusieurs chaînes latérales d'acides aminés naturels identiques ou différents ;
Y est choisi dans le groupe constitué d'une liaison, -C(=O)-NH-, -NH-C(=O)-, -NH-C(=S)-NH-; dans lequel le poids moléculaire Mₙ du copolymère semitéléchélique linéaire est compris entre 10 000 et 100 000 g/mol;
et dans lequel le fluorophore a un poids moléculaire compris entre 350 et 1 500 g/mol, une longueur d'onde d'excitation comprise entre 300 et 850 nm et une longueur d'onde d'émission comprise entre 350 et 1 200 nm, et est lié de manière covalente à l'unité monomère de formule générale (I) du copolymère semitéléchélique linéaire par l'intermédiaire d'une amine terminale ou d'un thiocyanate ou d'un ester *N*-hydroxysuccinimide, dans lequel les groupes formés après la liaison du fluorophore font ensuite partie du groupe Y;
et dans lequel le polymère fluorescent contient à une extrémité de la chaîne polymère un groupe fonctionnel pour la liaison à une structure protéique, lequel groupe fonctionnel est choisi dans le groupe comprenant les esters, de préférence l'ester de N-hydroxysuccinimide ou les esters d'alkyle (C1-C4), les amides, de préférence l'amide de thiozolidine-2-thione, le maléimide, l'azide, le propargyle, de préférence le groupe fonctionnel est l'azide ou le maléimide.

2. Le polymère fluorescent selon la revendication 1, dans lequel le copolymère statistique semitéléchélique linéaire comprend du poly(N-(2-hydroxypropyl)méthacrylamide, dans lequel de 0,4 à 12 % en moles d'unités monomères sont remplacées par des unités monomères de formule générale (I).

3. Le polymère fluorescent selon l'une quelconque des revendications 1 ou 2 précédentes, qui comprend en outre jusqu'à 11,6 % en moles d'unités monomères de formule générale (II), par rapport au nombre d'unités monomères, 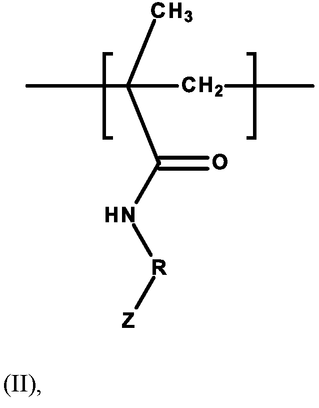 dans laquelle
R est tel que défini dans la revendication 1,
et Z est choisi dans le groupe comprenant -C(=O)-NH-(CH₂)ₐ-CH₂(OH) ; -C(=O)-NH-(CH₂)_{b}-CH(OH)-CH₃ ; -C(=O)-NH-(CH₂)_{b}-CH(OH)-(CH₂)_{c}-CH₃ ; où *a* est un entier de 0 à 4, *b* est un entier de 0 à 3 et *c* est compris entre 1 et 4 ; -NH-C(=O)-CH₃ ;
dans laquelle la contenu totale d'unités monomères de formule générale (I) et (II) dans le polymère fluorescent est d'au plus 12 % en moles, par rapport au nombre d'unités monomères.

4. Le polymère fluorescent selon l'une quelconque des revendications 1 à 3 précédentes, dans lequel le fluorophore est choisi dans le groupe comprenant la fluorescéine, l'isothiocyanate de fluorescéine, les dérivés de complexes ruthénium-bipyridine Ru(bpy)₃, en particulier le bis(2,2'-bipyridine)-[4-(4'-méthyl-2,2'-bipyridin-4-yl)butan-1-aminium ruthénium tris(perchlorate), les cyanines, 3,6-diamino-9-[4-(2-aminoéthylcarbamoyl)-2-carboxyphényl]-5-(3-sulfonatopropylsulfamoyl)xanthène-10-ium-4-sulfonate, (2E)-1-[6-(2,5-dioxopyrro-lidin-1-yl)oxy-6-oxohexyl]-2-[(2E,4E)-5-[1-(2-méthoxyéthyl)-3,3-diméthyl-5-sulfonato-indol-1-ium-2-yl]penta-2,4-diénylidène]-3,3-diméthyl-indoline-5-sulfonate, 3-[4-[(E)-2-[6-[6-(2-aminoéthylamino)-6-oxohexoxy]-1,3-benzoxazol-2-yl]vinyl]-1-pyridyl]propane-1-sulfonate, 3-[4-[6-[[6-(2,5-dioxopyrrolidin-1-yl)oxy-6-oxohexyl]carbamoylamino]-1,3-benzoxazol-2-yl]pyridin-1-ium-1-yl]propane-1-sulfonate, 3-[[6-(2-azaniumyl-éthylamino)-6-oxohexyl]carbamoyl]-8-chloro-7-hydroxy-2-oxo-chromène-6-sulfonate, 3-[4-[7-[[6-(2-aminoéthylamino)-6-oxohexyl]éthylamino]-2-oxo-chromen-3-yl]pyridin-1-ium-1-yl]propane-1-sulfonate, 1-[6-(2-aminoéthylamino)-6-oxo-hexyl]-6-[(E)-2-[7-(diéthylamino)-2-oxo-chromen-3-yl]vinyl]pyridin-1-ium-3-sulfonate, 1-[6-(2-aminoéthylamino)-6-oxo-hexyl]-4-[(E)-2-[7-(diéthylamino)-2-oxo-chromen-3-yl]vinyl]pyridin-1-ium-3-sulfonate, (2E)-2-[(E)-3-(7-amino-2-terc-butyl-chromenylium-4-yl)prop-2-énylidène]-1-[6-(2-aminoéthylamino)-6-oxohexyl]-3,3-diméthyl-indoline-5-sulfonate, (2Z)-3-[4-(2-aminoéthylamino)-4-oxo-butyl]-2-[(E)-3-[4-terc-butyl-7-[éthyl(3-sulfo-natopropyl)amino]chromenylium-2-yl]prop-2-énylidène]-3-méthyl-1-(3-sulfonatopropyl)indoline-5-sulfonate, et (2Z)-3-[4-(2-aminoéthylamino)-4-oxobutyl]-2-[(E)-3-[7-(diéthylamino)-3-méthyl-4-phényl-chroménylium-2-yl]prop-2-énylidène]-3-méthyl-1-(3-sulfo-natopropyl)indoline-5-sulfonate, ou éventuellement leurs dérivés contenant un groupe fonctionnel choisi parmi un groupe amino, un groupe isothiocyanate et un groupe N-hydroxysuccinimide.

5. Le polymère fluorescent selon l'une quelconque des revendications 1 à 4 précédentes, qui comprend au moins deux fluorophores différents.

6. Une sonde fluorescente comprenant au moins un polymère fluorescent selon l'une quelconque des revendications 1 à 5, auquel une structure protéique est liée de manière covalente via un groupe terminal de la chaîne polymère linéaire ;
dans lequel la structure protéique est choisie dans le groupe constitué par un peptide ayant de 10 à 200 acides aminés, une protéine, une lipoprotéine, un anticorps, une enzyme, une hormone ou un récepteur cellulaire ; de préférence, la structure protéique est un anticorps monoclonal, et le polymère fluorescent est lié de manière covalente à une chaîne légère de l'anticorps monoclonal, plus préférablement à une chaîne légère de l'anticorps monoclonal, choisie dans le groupe comprenant anti-CD20, anti-CD3, anti-CD4, anti-CD8, anti-CD71, anti-MHCII, anti-CD45, anti-JNK.

7. Un procédé de préparation de la sonde fluorescente selon la revendication 6, **caractérisé en ce qu'**il comprend les étapes suivantes :
a) Fournir les monomères du copolymère semitéléchélique linéaire, choisis dans le groupe comprenant le N-(2-hydroxypropyl)méthacrylamide, l'acrylamide, le méthacrylamide ;
et les monomères de formule générale (IV) 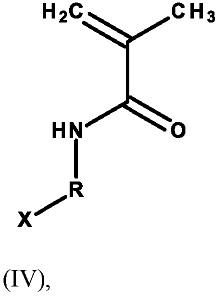 dans laquelle
R est choisi dans le groupe constitué par une chaîne alkylène carbonée linéaire ou ramifiée avec un nombre de carbones de 1 à 6 ; où R peut être en outre substitué par une ou plusieurs chaînes latérales d'acides aminés naturels identiques ou différents ;
et
X est un groupe carbonyl-thiazoline-2-thione ou un groupe -NH₂, où le groupe -NH₂ peut éventuellement être protégé par un groupe protecteur ;
b) Polymérisation des monomères entraînant la formation du copolymère semitéléchélique linéaire;
c) Liaison d'un fluorophore au copolymère semitéléchélique linéaire pour former un polymère fluorescent ;
d) Eventuellement, modification d'une structure protéique ;
e) Liaison de la structure protéique au polymère fluorescent.

8. Le procédé de préparation de la sonde fluorescente selon la revendication 7, **caractérisé en ce que** l'étape b) de polymérisation des monomères est réalisée par polymérisation radicalaire RAFT contrôlée des monomères de l'étape a) avec une contenu de 0,4 à 12 % en moles du monomère de formule générale (IV), et au moins 88 % en moles d'unités monomères choisies dans le groupe comprenant le N-(2-hydroxypropyl)méthacrylamide, l'acrylamide, le méthacrylamide ;
dans lequel la polymérisation est réalisée à une température comprise entre 30 et 100 °C, et un solvant choisi de préférence dans le groupe comprenant le diméthylsulfoxyde, le diméthylacétamide, le diméthylformamide, le méthanol, l'éthanol, le dioxane, le tétrahydrofurane, le propanol, le *tert*-butanol ou leurs mélanges ;
la réaction est initiée par un initiateur, de préférence choisi dans le groupe comprenant la 2,2'-azobis[N-(2-carboxyéthyl)-2-méthylpropionamidine] et la N-(3-azidopropyl)-4-[(Z)-[4-(3-azidopropylamino)-1-cyano-1-méthyl-4-oxobutyl] azo]-4-cyano-pentanamide ;
éventuellement en présence d'un agent de transfert, de préférence la N-(3-azidopropyl)-4-éthylsulfanylcarbothioylsulfanyl-4-méthyl-pentanamide ;
éventuellement suivie de l'élimination des groupes protecteurs protégeant les groupes amine des chaînes latérales.

9. Le procédé de préparation de la sonde fluorescente selon la revendication 7 ou 8, **caractérisé en ce que** l'étape c) de liaison d'un fluorophore au copolymère semitéléchélique linéaire est réalisée par conjugaison des groupes amine libres ou des groupes carbonyl-thiazolidine-2-thione des unités monomères de formule générale (IV) du copolymère semitéléchélique linéaire à un fluorophore, qui contient un groupe amine réactif ou SCN,
dans lequel le fluorophore a un poids moléculaire dans la plage de 350 à 1 500 g/mol, les longueurs d'onde d'excitation dans la plage de 300 à 850 nm et les longueurs d'onde d'émission dans la plage de 350 à 1 200 nm ;
et dans lequel le fluorophore est lié au copolymère semitéléchélique linéaire par une liaison amide ou thioamide ;
éventuellement, les groupes thiazolidine-2-thione n'ayant pas réagi sont éliminés par réaction avec un aminoalcool, choisi dans le groupe comprenant NH₂-(CH₂)ₐ-CH₂(OH) ; NH₂-(CH₂)_{b}-CH(OH)-CH₃ ; NH₂-(CH₂)_{b}-CH(OH)-(CH₂)_{c}-CH₃ ; où *a* est un entier de 0 à 4, *b* est un entier de 0 à 3 et c est de 1 à 4, et/ou les groupes -NH₂ n'ayant pas réagi sont éliminés par réaction avec de l'acétylthiazolidine-2-thione ;
éventuellement, le polymère fluorescent résultant est soumis à d'autres réactions conduisant à un changement du groupe réactif à l'extrémité de la chaîne semitéléchélique, de préférence à une réaction de clic d'un groupe azide avec du dibenzocyclooctyne maléimide pour former un groupe fonctionnel maléimide à une extrémité de la chaîne polymère linéaire ;
ou à une réaction de clic d'un groupe azoture avec de l'acide dibenzocyclooctyne-carboxylique puis avec de la thiazolidine-2-thione pour former un polymère fluorescent avec un groupe fonctionnel thiazolidine-2-thione à une extrémité de la chaîne polymère linéaire ;
ou à une réaction de clic d'un groupe azoture avec un ester dibenzocyclooctyne-N-hydroxysuccinimide pour former un polymère fluorescent avec un groupe fonctionnel ester N-hydroxysuccinimide à une extrémité de la chaîne polymère linéaire.

10. Le procédé de préparation de la sonde fluorescente selon l'une quelconque des revendications 7, 8 ou 9, **caractérisé en ce que** l'étape d) de modification d'une structure protéique est réalisée en réduisant les liaisons disulfures de la structure protéique avec un agent réducteur, choisi dans le groupe du dithiothréitol, de la tris(2-carboxyéthyl)phosphine, du *β*-mercaptoéthanol, du glutathion ; dans un tampon aqueux à pH de 6,5 à 7,5 et à température ambiante, pour former des groupes
-SH dans la structure protéique, qui peuvent éventuellement être modifiés davantage par réaction avec du sulfodibenzocyclooctyne maléimide pour former des groupes sulfodibenzocyclooctyne ; et/ou les groupes NH₂ libres de la structure protéique sont modifiés en groupes dibenzocyclooctyne par une réaction avec l'ester dibenzocyclooctyne-*N*-hydroxysuccinimide.

11. Le procédé de préparation de la sonde fluorescente selon l'une quelconque des revendications 7, 8, 9 ou 10, **caractérisé en ce que** l'étape e) de liaison de la structure protéique au polymère fluorescent est réalisée par conjugaison d'au moins un polymère fluorescent selon l'une quelconque des revendications 1 à 5 ; et de la structure protéique, éventuellement de la structure protéique avec des groupes sulfhydryle, sulfodibenzocyclooctyne ou dibenzocyclooctyne introduits préparés à l'étape d),
la réaction ayant lieu à température ambiante dans un tampon aqueux et à un pH de 6,5 à 7,5.

12. Un kit de conjugaison pour le marquage d'anticorps, **caractérisé en ce qu'**il comprend le polymère fluorescent selon l'une quelconque des revendications 1 à 5 ; un tampon de réaction de pH 7, comprenant 0,1 M de tampon phosphate et 10 mM d'EDTA ; du dithiothréitol ; et au moins une colonne ayant un volume compris entre 0,5 et 2 ml, qui contient du dextrane réticulé ayant un poids moléculaire compris entre 100 et 5 000 g/mol.

13. Utilisation du polymère fluorescent selon l'une quelconque des revendications 1 à 5 et/ou du kit de conjugaison selon la revendication 12, pour le marquage fluorescent de structures protéiques, choisies dans le groupe constitué par un peptide, une protéine, une lipoprotéine, un anticorps, une enzyme, une hormone, un récepteur, une protéine structurale, une protéine de transport, une protéine de voie de signalisation cellulaire, une protéine synthétique.

14. Utilisation du polymère fluorescent selon l'une quelconque des revendications 1 à 5 et/ou de la sonde fluorescente selon la revendication 6, dans des techniques d'imagerie fluorescente, de préférence choisies dans le groupe comprenant la cytométrie de flux, les dosages par billes, la microscopie, le western blot, la cytométrie de flux par fluorescence.

15. La sonde fluorescente selon la revendication 6 pour l'utilisation comme agent de contraste dans le diagnostic médical, l'imagerie du corps entier et/ou la chirurgie assistée par fluorescence, de préférence dans le diagnostic du cancer, des maladies inflammatoires et bactériennes.
